# EUROPEAN PATENT SPECIFICATION

(11) **EP 3 052 177 B1**
(45) Date of publication and mention of the grant of the patent: **01.04.2020**
(21) Application number: 14792920.2
(22) Date of filing: 30.09.2014
(51) Int. Cl.: A61M 25/09

(54) **GUIDE WIRE CORE WITH IMPROVED TORSIONAL DUCTILITY**
FÜHRUNGSDRAHTKERN MIT VERBESSERTER TORSIONSDUKTILITÄT
C UR DE FIL-GUIDE POSSÉDANT UNE DUCTILITÉ EN TORSION AMÉLIORÉE

(30) Priority: 30.09.2013 US 201314042321; 29.09.2014 US 201414499856
(43) Date of publication of application: 10.08.2016
(73) Proprietor: Abbott Cardiovascular Systems, Inc., Santa Clara, CA 95054 (US)
(72) Inventor: SIMPSON, John A., Carlsbad, California 92009 (US); DOOLEY, Jeffrey F., Oceanside, California 92056 (US); PURTZER, Raleigh A., Temecula, California 92591 (US); GILLICK, Matthew J., Murrieta, California 92562 (US)
(74) Representative: Zacco GmbH
(86) International application number: PCT/US2014/058320
(87) International publication number: WO 2015/048750

(56) References cited:
- EP-A1- 0 395 098
- EP-A1- 1 388 350
- EP-A2- 0 519 604
- EP-A2- 0 806 220
- WO-A1-2010/107798
- WO-A1-2012/058302
- WO-A1-2013/114985
- WO-A2-94/20165
- WO-A2-99/42158
- WO-A2-2006/002199
- US-B1- 6 736 843
- US-B1- 8 100 837

## Description

### BACKGROUND

The present application relates generally to guide wires for intraluminal application in medical procedures, and methods for their manufacture. More specifically, the present application relates to guide wires that possess varying properties of flexibility and torsional stiffness along their length, particularly possessing varying flexibility and torsional stiffness characteristics along an extreme distal tip portion of the guide wire.

The human body includes various lumens, such as blood vessels or other passageways. Guide wires have been used in the art of minimally invasive medical procedures, e.g., in conjunction with catheters used to access various locations within the body. For example, a placement catheter may be threaded into a desired body lumen, and a guide wire inserted through the catheter into the body lumen. Thereafter, the practitioner may use the guide wire as various catheters, instruments, or other devices are placed and withdrawn, using the guide wire as a guide. For example, a stent or other intracorporal device may be introduced into a desired position using such techniques.

For example, a lumen within the placement catheter permits the physician to insert a guide wire through the catheter to the same location. Thereafter, when the physician may need to sequentially place a second, or third, or even a fourth catheter to the same location, it is a simple matter to withdraw the catheter while leaving the guide wire in place. After this action, second, third, and fourth etc. catheters may be sequentially introduced and withdrawn over the guide wire that was left in place. In other techniques, a guide wire may be introduced into the vasculature of a patient without the assistance of a placement catheter, and once in position, catheters may be sequentially inserted over the guide wire as desired.

It is typical that best medical practice for anatomical insertion in at least some circumstances requires a guide wire that has behavioral characteristics that vary along its length. For example, under some conditions, the distal end of the guide wire may he required to be more flexible than the proximal end so that the distal end may more easily be threaded around the more tortuous distal branches of the luminal anatomy. Further, the proximal end of the guide wire may be required to have greater torsional stiffness than the distal end because, upon rotation of the guide wire, the proximal end must carry all the torsional forces that are transmitted down the length of the guide wire from the distal end, whereas the distal end must transmit only those torsional forces that are imparted locally.

Finally the distal end of a guide wire should be selectively formable, so that the treating physician may apply a curve to the tip of the catheter in order to facilitate navigation along the tortuous passageways of the vascular anatomy. By selectively formable, it is meant that the wire from which the guide wire core is made may be bent to a particular shape and that the shape will be maintained by the wire. This allows the physician to impart a particular shape to the guide wire, by bending or kinking it for example, to facilitate steering its placement into a patient's vasculature. To provide this selective formability, in typical embodiments, the entire core wire may be made of stainless steel. However, other materials may be used to provide this feature. The use of a formable material, such as stainless steel, provides advantages in the guide wire over materials that cannot be formed, such as superelastic materials like nitinol. Superelastic materials like nitinol are so resilient that they tend to spring back to their original shape even if bent, thus are not so readily deformable. Although superelastic material may be provided with a "preformed" memory shape, such a preformed shape is typically determined in the manufacture of the guide wire and cannot readily be altered or modified by the physician by simply bending the guide wire prior to use. Although use of superelastic materials such as nitinol in guide wire applications may provide some advantages in certain uses, a formable core, such as of stainless steel, which can be formed by the physician to a shape suitable for a particular patient or preferred by that physician, provides an advantage that cannot be obtained with a superelastic core guide wire.

WO2010/107799 A1 discloses a guidewire with a guidewire tip portion wherein the proximal tip has a circular cross section and exhibits superelastic properties.
WO2006/002199 A2 discloses a method for manufacturing a tip portion of a guide wire.

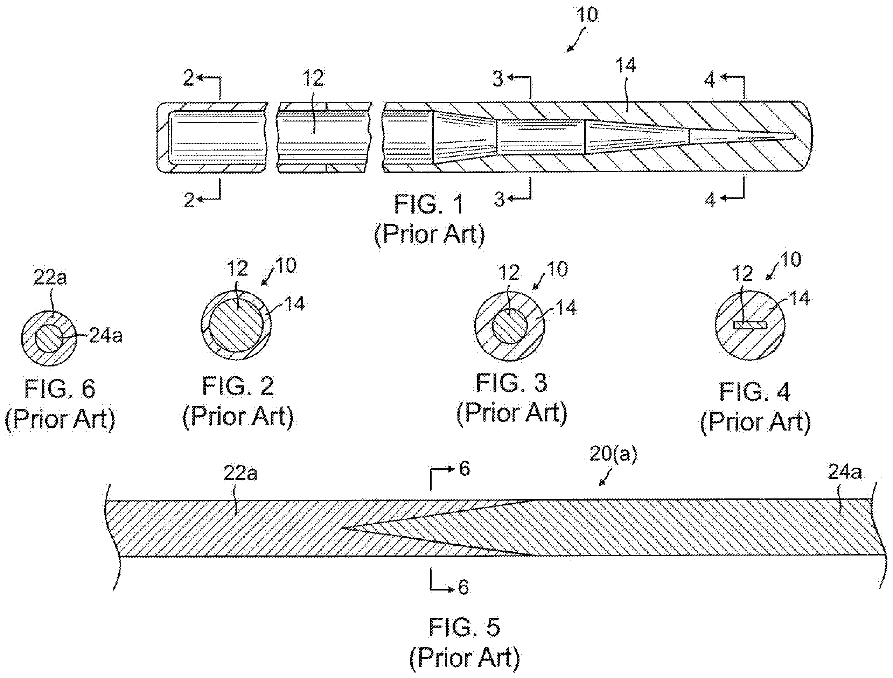

Thus, certain solutions have been developed in the art to address these requirements. In one typical solution, a guide wire may be fabricated by applying the same metallurgical process along the entire length of an initial ingot of uniform metallurgical properties and uniform diameter that will be converted into the guide wire. The initial ingot may be taken up and cold worked along its entire length, or annealed, or whatever process is required to impart the desired characteristics to the metal of the final guide wire product. Once these metallurgical processes have been performed on the wire as a whole, the wire obtained from the worked ingot may be geometrically shaped in order to impart desired different flexibilities, torsional stiffnesses and the like that are desired in the final guide wire product. For example, a worked ingot may be shaped by known process such as chemical washes, polishes, grinding, or compressing, to have a distal end with a diameter that is smaller than the diameter of the proximal end. By this means, the distal end will be given greater flexibility but less torsional resistance than the proximal end. A shaped guide wire 10 of the kind described is depicted in Figure 1 where it may be seen that a core metal element 12 having a configuration with varying diameter sizes along its length is coated in a polymer 14, or other suitable material to add lubricity. The coating may be configured to impart a uniform outside diameter to the overall guide wire 10.

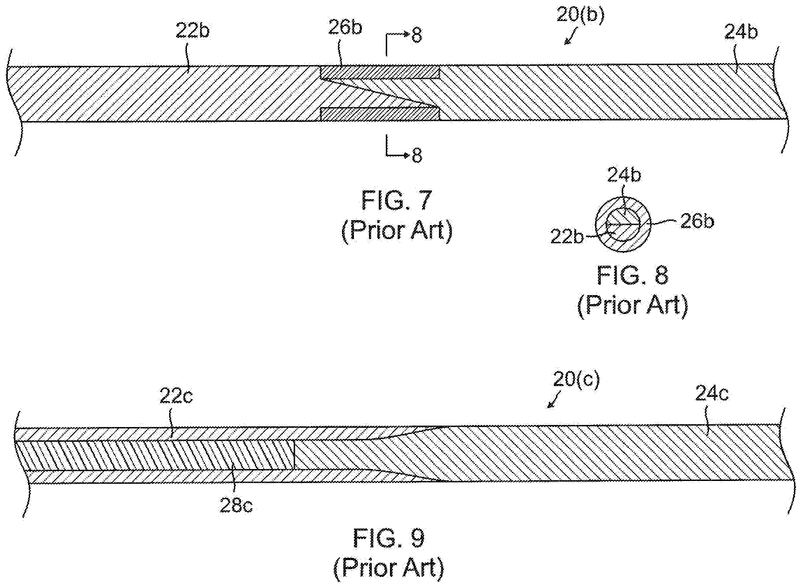

In another typical solution, different pieces of wire may be formed by different processes to have different properties. These pieces of wire may then be joined or connected together into a single guide wire core using known jointing processes, to provide a resulting guide wire with varying properties along its length. For example, as may be envisaged with reference to Figure 5 through Figure 9, different embodiments 20a, 20b, and 20c show how a superelastic portion of wire 22a, 22b, and 22c made from nitinol or similar metal, may be joined to a portion of wire 24a, 24b, and 24c that has linear elastic properties using jointing methods such as welding, or covering with a jacket 26b, or inserting a filler 28c.

Thus, in a core wire having this combination of a distinct and joined formable distal portion and a superelastic proximal portion, desired shapes may be imparted by a physician to the distal end of the guide wire to facilitate making turns, etc., in tortuous vessel passages, while in the same guide wire the more proximal portion would possess superelastic properties to allow it to follow the distal portion through the tortuous passages without permanently deforming.

However, problems may arise in the art as described. Welds or other joints are generally undesirable on a guide wire because they introduce a potential point of kinking or fracture. Furthermore, discrete steps in the gradient of a guide wire diameter that are introduced by grinding or other known means may also introduce potential points at which stress is raised to produce cracking or fracture.

For example, guide wires may often include an elongate core member with one or more segments near the distal end where the segments taper distally to smaller cross-sections. The proximal portion of the elongate core member may be relatively stiff, e.g., to provide the ability to support a balloon catheter or similar device. The distal portion may be increasingly flexible, with moderate flexibility adjacent the stiffer proximal portion, and becoming increasingly flexible towards the distal end. For example, the distal portion may be formed of a super-elastic alloy, such as nitinol. A relatively short section of the extreme distal end of the core tip may be flattened to impart cold work thereto, altering its material properties to make the extreme distal tip of the core wire easier to shape. For example, this may allow a practitioner to impart a J, L, or similar bend to the flattened distal tip, e.g., by deforming the extreme distal tip through finger pressure. Such a bent tip may be advantageous for steering through a patient's vasculature.

Despite a number of different available guide wire devices, and related methods of manufacture, there still remains a need for improved guide wires and associated methods of manufacture.

### BRIEF SUMMARY

In some embodiments, the invention is a method for making a core metal element for a medical guide wire. The method comprises providing a wire of nickel titanium alloy having a length that includes a proximal portion having a first diameter and a distal portion having a second diameter. In some embodiments, the first diameter may be the same as the second diameter. Once a suitable length of wire is selected, cold work is applied to the distal portion, while little or no cold work is applied to the proximal portion. By this action, there is imparted to the distal portion a third diameter that is smaller than the second diameter. In other words, the diameter of the distal portion is slightly diminished by the application of cold work. Thereafter, a reducing process is applied to the wire whereby the proximal portion is reduced to have a fourth diameter that is less than the first diameter. By this process, the reducing process may diminish the larger diameter of the proximal portion. The reducing process may stop when the diameters of the proximal portion and the distal portion are initially the same, or, in other words, when the fourth diameter is the same as the third diameter. Or, the reducing process may continue to diminish the diameters of both the proximal and the distal portions, such that they each have a fifth diameter that is smaller than the third diameter.

In some embodiments, the step of providing a wire includes providing a wire with superelastic properties throughout the length, and the step of applying cold work to the distal portion includes applying sufficient cold work to render the distal portion to have linear elastic properties. By imparting linear elastic properties to the distal portion, that portion becomes formable by the physician. Furthermore, after applying cold work to the distal portion, the proximal portion retains its original superelastic properties as no significant cold work has been applied to that portion. Notably, no welding process may be applied to the wire over the length, and no joint is necessarily created or inserted into the wire over the length.

In some embodiments, the step of applying a reducing process to the guide wire includes applying centerless grinding. In other embodiments the step of applying a reducing process includes chemical wash or electrochemical removal, or an electrochemical or mechanical polishing process.

In some embodiments the step of applying cold work to the distal portion includes drawing the distal portion through a die, and in further embodiments the guide wire may be removed from the die without drawing the distal portion back through the die. In other embodiments, the step of applying cold work to the distal portion includes applying cold work methods selected from: swaging, tensioning, rolling, stamping, and coining.

In some embodiments, the step of providing a wire includes providing a wire wherein the proximal portion is adjacent the distal portion.

In some embodiments, the step of providing a wire includes providing a wire wherein the proximal portion is adjacent a proximal end of the wire, or, wherein the distal portion is adjacent a distal end of the wire.

In some embodiments, the invention is a medical guide wire comprising a solid metal core having a length and having a substantially constant diameter over the length, wherein the length includes a proximal portion having pseudoelastic properties (interchangeably referred to herein as superelastic properties) and a distal portion having linear elastic properties. The length of the core may not include a mechanical joint at any location situated between the proximal portion and the distal portion. The length of the core also may not include a metallurgical joint, such as a solder, braze, or weld joint, at any location situated between the proximal portion and the distal portion. In further embodiments, the proximal portion is formed from a nickel titanium alloy (e.g., nitinol), and in yet further embodiments, the distal portion includes metal to which the linear elastic properties have been imparted by a process of cold working.

In another embodiment, the present disclosure describes methods for manufacturing a tip portion of a guide wire. The method may include providing a superelastic wire (e.g., nitinol) including a length so as to define both a distal tip portion and a proximal tip portion. The distal tip portion is cold worked, without imparting significant cold work to the proximal tip portion, so as to provide linear elastic, rather than superelastic properties within the distal tip portion, so that the proximal tip portion exhibits superelastic properties and the distal tip portion exhibits linear elastic properties. The tip portion is ground or otherwise reduced in cross-sectional thickness after cold working of the distal tip portion, so as to provide a circular cross-section having a desired substantially constant diameter along the distal tip portion and the proximal tip portions of the tip.

Such methods advantageously result in a distal tip portion, which can accommodate a bend (J-bend, L-bend, or other) by the practitioner, but in which the cross-section of the distal tip portion remains circular, and of substantially the same diameter as the adjacent proximal tip portion, so that the entire tip portion of the guide wire has substantially the same diameter along the entire tip portion, and may comprise a single piece of material, without any mechanical joint between the linear elastic distal tip portion and the superelastic proximal tip portion. Such a circular guide wire tip advantageously provides smooth torque response, rather than exhibiting a tendency to "whip" as a practitioner applies torque to the guide wire. For example, a non-circular tip (e.g., rectangular, such as results by flattening) may tend to pause as torsion builds up in the guide wire, until a threshold level or torsion builds up, at which point it abruptly whips around (e.g., a half turn), pausing again until another threshold level of torsion builds up. Such whipping is undesirable as it may diminish the control achievable by the practitioner during guide wire manipulation.

According to another embodiment, a method of manufacture may include providing a superelastic wire including a length so as to define both a distal tip portion and a proximal tip portion. The distal tip portion is subjected to rotary swaging without imparting significant cold work to the proximal tip portion to provide linear elastic rather than superelastic properties to the distal tip portion, so that the proximal tip portion exhibits superelastic properties and the distal tip portion exhibits linear elastic properties. At least part of the tip portion is ground after cold working of the distal tip portion to provide a circular cross-section having a desired substantially constant diameter along the distal tip portion and the proximal tip portion of the tip. The distal tip portion may have a length that is about 30% to about 70% that of a combined length of the distal tip portion and the proximal tip portion.

Another embodiment is directed to a guide wire including a guide wire core tip portion including a distal tip portion and a proximal tip portion. The tip portion includes a substantially constant diameter along both the distal tip portion and the proximal tip portion. The distal tip portion may have a circular cross-section and exhibit linear elastic properties, while the proximal tip portion also has a circular cross-section, substantially the same diameter as the distal tip portion, but exhibits superelastic properties.

These and other objects and features of the present disclosure will become more fully apparent from the following description and appended claims, or may be learned by the practice of the embodiments of the invention as set forth hereinafter.

### BRIEF DESCRIPTION OF THE DRAWINGS

To further clarify the above and other advantages and features of the present disclosure, a more particular description of the invention will be rendered by reference to specific embodiments thereof which are illustrated in the appended drawings. It is appreciated that these drawings depict only illustrated embodiments of the invention and are therefore not to be considered limiting of its scope. Embodiments of the invention will be described and explained with additional specificity and detail through the use of the accompanying drawings in which:
Figure 1 shows a partial sectional view of a prior art guide wire with a sequence of diameter reductions, shown in shortened schematic form.
Figure 2 is a sectional view through the guide wire of Figure 1, taken substantially along the line 2-2 in Figure 1.
Figure 3 is a sectional view through the guide wire of Figure 1, taken substantially along the line 3-3 in Figure 1.
Figure 4 is a sectional view through the guide wire of Figure 1, taken substantially along the line 4-4 in Figure 1.
Figure 5 shows a sectional view of a prior art guide wire with proximal and distal portions joined together.
Figure 6 is a sectional view through the guide wire of Figure 5, taken substantially along the line 6-6 in Figure 5.
Figure 7 shows a sectional view of a prior art guide wire with proximal and distal portions joined together.
Figure 8 is a sectional view through the guide wire of Figure 7, taken substantially along the line 8-8 in Figure 7.
Figure 9 shows a sectional view of a prior art guide wire with proximal and distal portions joined together.
Figure 10 is a schematic side view of a wire in a first condition in a process of preparation for use according to an embodiment of the present invention.
Figure 11 is a schematic side view of a wire in a second condition in a process of preparation for use according to an embodiment of the present invention.
Figure 12 is a schematic side view of a wire in a third condition in a process of preparation for use according to an embodiment of the present invention.
Figure 13 is a schematic side view of a wire in a fourth condition in a process of preparation for use according to an embodiment of the present invention.
Figure 14 is a schematic side view of a wire in a fifth condition in a process of preparation for use according to an embodiment of the present invention.
Figure 15A is a side elevation and partial cross-sectional view of an exemplary intraluminal guide wire according to an embodiment of the present disclosure.
Figure 15B is a close up view showing the tip portion of the guide wire of Figure 15A.
Figure 16 is a side elevation view of another intraluminal guide wire according to an embodiment of the present disclosure.
Figure 17A is a flow chart illustrating a method for manufacturing an intravascular guide wire according to an embodiment of the present disclosure.
Figure 17B is a flow chart illustrating another method for manufacturing an intravascular guide wire according to an embodiment of the present disclosure.
Figure 18 is a schematic side view of a superelastic wire including a length sufficient to define both a distal tip portion and a proximal tip portion.
Figure 19 is a schematic side view of the wire of Figure 18, the distal portion thereof having been cold worked to provide linear elastic properties therein.
Figure 20A is a schematic view of the wire of Figure 19, the proximal tip portion thereof having been reduced in cross-sectional thickness so as to provide a circular cross section and substantially constant diameter across both the superelastic proximal tip portion and the linear elastic distal tip portion.
Figure 20B is a schematic view of the wire of Figure 19, both the proximal tip and distal tip portions thereof having been reduced in cross-sectional thickness so as to provide a circular cross section and substantially constant diameter across both the superelastic proximal tip portion and the linear elastic distal tip portion.
Figure 21 is a schematic view of a wire of Figures 20A or 20B, wherein a coating or other exterior layer has been applied over at least part of the tip portion.
Figure 22 is a close up view showing an exemplary tip portion of a guide wire, similar to that of Figure 15B.

### DETAILED DESCRIPTION

In conjunction with the figures, there is described herein a medical guide wire and a method for manufacturing a medical guide wire having features of an embodiment of the present invention. In some embodiments, the invention includes a method for forming a core for a guide wire of an embodiment according to the present invention.

In its final form, the guide wire may comprise an elongated solid core wire 112 and an outer jacket 114 made from a polymer with lubricious, or with hydrophilic or even with hydrophobic qualities, depending on the needs of the situation. The elongated solid core wire 112 includes a proximal section 116 of a constant diameter, and a distal section 118.

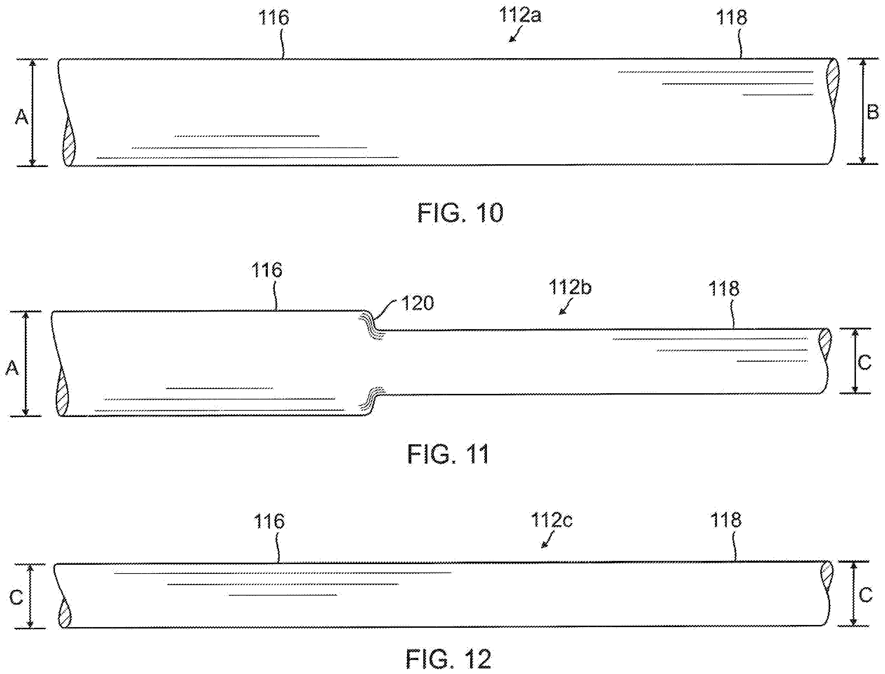

The core wire may preferably be made of a NiTi alloy. In some embodiments, the NiTi alloy useful for the present invention may be initiated by preparing an ingot which is melted and cast using a vacuum induction or vacuum arc melting process. The ingot is then forged, rolled and drawn into a wire. In some embodiments, exemplified in Figure 10, the resulting core wire 112a may have a diameter of about 0.030 inches in diameter, and may have a nominal composition of about 55.0 weight percent Ni and an austenite transformation start (As) temperature of about 0°C in the fully annealed state. In this form, the wire may exhibit superelastic properties at a body temperature of about 37°C, which are desirable in at least portions of a guide wire so that those portions do not permanently deform as they are extended through a tortuous anatomy.

Once the initial basic wire 112a has been thus prepared, a length of wire that is desired to possess linear elastic properties is identified and selected. With reference to Figures 11 to 14, this selected length is identified by the reference numeral 118 and is referred to herein as the distal portion of the wire. A portion of the wire that is not desired to possess linear elastic properties, but to retain its superelastic properties, is identified by the numeral 116 and is referred to herein as the proximal portion 111. In some embodiments, the proximal portion 116 and the distal portion 118 are selected to be adjacent to each other, but this is not a limiting requirement of at least some embodiments of the invention. In fact, portions of the wire between the proximal portion 116 and the distal portion 118 may be selected for yet further and different treatment than that set forth herein below. In this initial condition, the wire is configured so that the proximal portion has a diameter "A," and the distal portion may have a second diameter "B" as shown in Figure 10. In some embodiments, the first diameter A is the same as the second diameter B, while in other embodiments these diameters may purposely differ and may have a gradual taper between them.

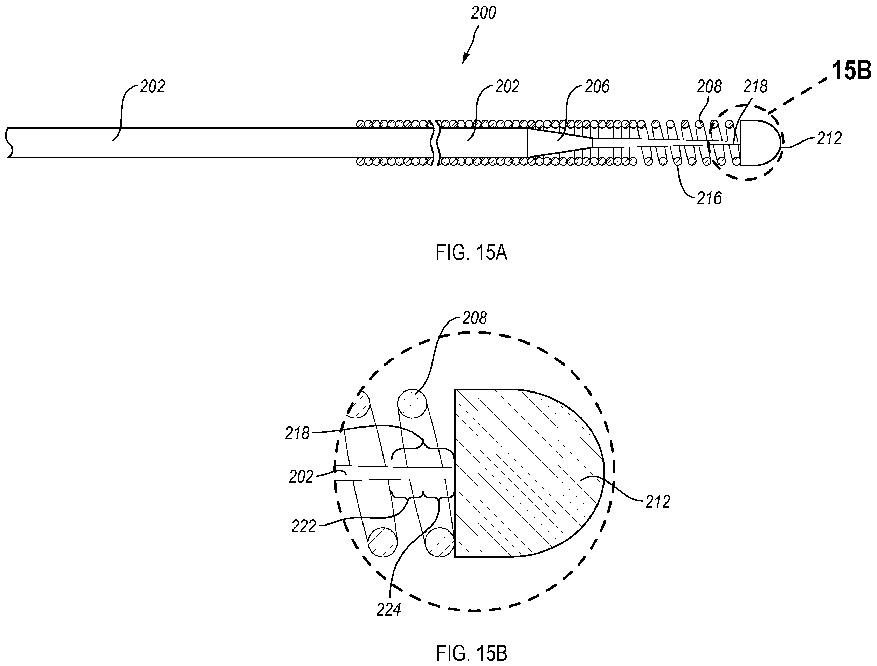

In either case, the following manufacturing steps may be performed. Cold work may be applied to the distal portion 118 of the wire, without applying cold work to the proximal portion 116 of the wire. By applying cold work to the distal portion 118, the diameter of the distal portion is given a third diameter "C" that is less than the second diameter "B", as seen in Figure 11. In some embodiments, the cold work may be applied by drawing the distal portion through a die and then removing it by reverse drawing. This overall process may further include removing the wire from the die without drawing the distal portion 118 back through the die, such as by using a multiple-piece die which can be opened to enable wire removal. In other embodiments, applying cold work to the distal portion may include methods selected from swaging, tensioning, rolling, stamping, and coining. In some embodiments, swaging may utilize a set of two or more revolving dies which radially deform the workpiece repeatedly as it passes between the dies. Like wiredrawing, swaging can produce an essentially round cross-section of reduced diameter. However the resulting work hardening is typically non-uniform across its final cross-section due to the so-called "redundant work" caused by repeated re-ovalization as the revolving dies repeatedly strike the non-revolving workpiece (which may be in 60° increments, in some embodiments). The final distribution of cold work may be influenced by both feed rate and die strike rate, and likely also by the contact length of the die set. Hence, judicious selection of processing conditions is required to attain the desired level of cold work within the distal section of the nitinol core wire before grinding to final size. Additional details of a rotary swaging process as described below, in conjunction with Figures 15A-22.

Regardless of initial straightness of a wire, it is typical for as-drawn wire to become curved as a result of passing through a wiredrawing die. This can be remedied by simultaneously applying heat and tension to induce stress relaxation within the as-drawn portion. This straightening method can be applied to the present invention, provided the time and temperature are not sufficient to restore original superelastic properties, which typically takes several minutes at about 500°C. A suitable combination of tension and heat may be determined through experimentation, with the goal of attaining suitable straightness for a drawn portion, which persists after producing the final guide wire core profile.

Once the wire is given satisfactory metallurgical properties by differential treatments such as those described, it will be appreciated that the wire may have a stepped shoulder 120 as exemplified by wire 112b seen in Figure 11, where the distal portion 118 may have linear elastic properties, and the proximal portion 116 may retain the original superelastic properties inherent in the unworked nickel titanium alloy. It will be appreciated that the step 120 seen in Figure 11 may have a steep stepped gradient, or a more gently sloping gradient, depending on the precise process by which cold work is applied to the distal portion 118.

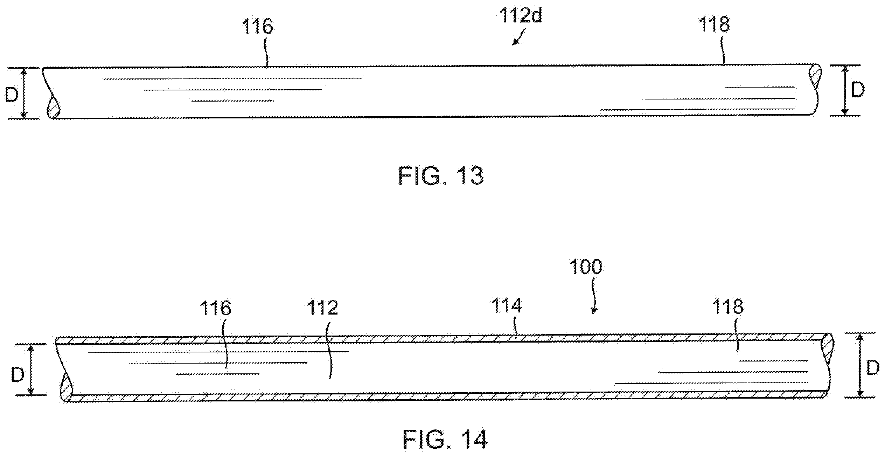

In a subsequent stage, the wire may then be subjected to a reducing process, in which the step 120, (i.e., the differential diameter between the proximal portion 116 and the distal portion 118) is removed. In this stage, the step 120 may be removed to impart the proximal portion 116 of the wire 112c to have a diameter "C" that is the same as the existing third diameter "C" of the distal portion 118, as seen in Figure 12. Alternatively, the wire 112d may be further reduced so that both proximal and distal portions are reduced so that each has a fourth diameter "D" that is smaller than diameter "C", as seen in Figure 13.

In some embodiments, the process of reducing the wire may be centerless grinding, which is a machining process that uses abrasive cutting to remove material from a workpiece. In some forms of centerless grinding, the workpiece is held between a workholding platform and two wheels rotating in the same direction at different speeds. One wheel, known as the regulating wheel, is on a fixed axis and rotates such that the force applied to the workpiece is directed downward, against the workholding platform. This wheel usually imparts rotation to the workpiece by having a higher linear speed than the other wheel. The other wheel, known as the grinding wheel, is movable. This wheel is positioned to apply lateral pressure to the workpiece, and usually has either a very rough or a rubber-bonded abrasive to grind away material from the workpiece. The speed of the two wheels relative to each other provides the rotating action and determines the rate at which material is removed from the workpiece by the grinding wheel. During operation the workpiece turns with the regulating wheel, with the same linear velocity at the point of contact and (ideally) no slipping. The grinding wheel turns faster, slipping past the surface of the workpiece at the point of contact and removing chips of material as it passes. In other embodiments of the invention, the reducing process may include chemical washes, or polishes.

Once these reducing steps as described above are performed, the wire 112c or 112d will have a uniform diameter "C" or "D" respectively throughout the proximal portion and distal portion. It will be appreciated however that, despite its uniform geometrical shape the wire will have differential metallurgical properties in the proximal and distal portions, and hence differential flexural and torsional stiffnesses and also deformation related properties.

In another aspect, the present disclosure describes guide wires, as well as methods for manufacturing the tip portion of a guide wire in order to provide linear elastic properties to the distal portion of the guide wire tip, while providing superelastic properties to the proximal portion of the guide wire tip. This can advantageously be achieved while providing a circular cross-section and substantially constant diameter along the entire length of the tip portion of the guide wire, reducing or eliminating any tendency of the guide wire tip to "whip" during torsion or twisting. Advantageously, this may be provided in a guide wire tip portion which is formed from an integral single piece of material (e.g., a nitinol wire), where the distal tip portion is cold worked in a manner that maintains its circular cross-section within the final product while rendering it linear elastic, rather than superelastic. Thus, the distal tip portion may be linear elastic nitinol, so as to readily accept a J-bend, L-bend, or other bend desired by the practitioner, while the proximal tip portion may be superelastic nitinol, so as to yield more readily and endure greater torsional deformation than the linear elastic distal tip portion. As a result, such a product minimizes or eliminates whipping characteristics, while providing relatively greater durability (e.g., it may exhibit higher durability in terms of turns to failure) to the tip of the guide wire.

Such guide wires may be manufactured by providing a superelastic wire including a length defining both a distal tip portion and a proximal tip portion, by cold working the distal tip portion without imparting significant cold work to the proximal tip portion, and by grinding or otherwise reducing the cross-sectional thickness of the tip portion after cold working to provide a circular cross-section and substantially constant diameter along the entire tip portion - i.e., both the distal tip portion and the proximal tip portion.

Figure 15A is an elevation side view and partial cross-sectional view of a guide wire 200 including features according to the present disclosure. Guide wire 200 may be adapted for insertion into a body lumen of a patient, for example a vein or artery. Guide wire 200 may include an elongate, relatively high strength proximal core portion 202. Core portion 202 may sometimes be provided by joining two different materials together, so as to provide a proximal portion of higher strength and stiffness, and a distal portion that may provide increased flexibility. For example, a proximal portion of a guide wire core may be formed of stainless steel, while the distal portion may be formed of nitinol. Joining of two such different materials may be achieved by any suitable technique. Of course, in other embodiments, a guide wire may be formed of a single material throughout the core, as desired. In any case, as one approaches the distal extreme end of the guide wire core, a tapered section 206 may be provided, tapering to a smaller thickness in the distal direction. A helical coil 208 may be disposed about a distal portion of core 202, while a rounded plug 212 (e.g., a solder tip) may be provided at the distal end.

As shown, a distal section 216 of coil 208 may be stretched in length to provide additional flexibility. Tip portion 218 of core 202 may be formed as described herein. For example, rather than flattening tip 218 to include a rectangular cross-section and render it capable of accepting a bend, it may be provided with proximal and distal tip portions as described herein having different properties, but including substantially the same circular cross-section and diameter for improved torsional control and durability.

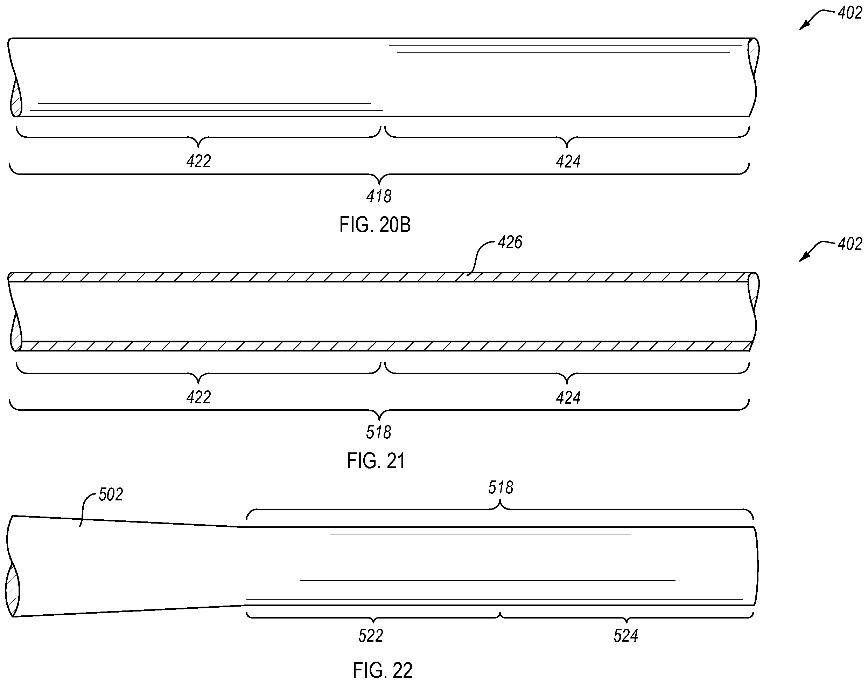

Figure 15B shows a close up view of tip portion 218, illustrating how it includes a proximal tip portion 222 and a distal tip portion 224. As is apparent in close up view of Figure 15B (and even more so in Figure 22), the diameter of tip portion 218, including both portions 222 and 224 is substantially constant, such that any taper that was present in the adjacent further proximal section of core 202 ends or substantially ends at the start of proximal tip portion 222 of tip portion 218. Providing tip portion 218 with a substantially constant diameter along its length advantageously provides an extreme distal tip of the core wire 202 that exhibits moment of inertia characteristics, which depend heavily on diameter, that are consistent within the smallest substantially constant diameter tip portion 218. The diameter within tip portion 218 may be from about 0.001 inch to about 0.005 inch, from about 0.001 inch to about 0.004 inch, from about 0.015 inch to about 0.0035 inch, or from about 0.002 inch to about 0.003 inch.

By substantially constant, it is meant that the diameter of the tip portion is either actually constant in diameter, or it may include a very shallow taper (e.g., tapered towards the distal tip portion). Such a shallow taper would be sufficiently shallow to still allow the torsional deformation to preferentially occur within the superelastic proximal tip portion of the guide wire tip. By way of example, such a taper may be less than about 10%, less than about 9%, less than about 8%, less than about 7%, less than about 6%, about 5%, less than about 4%, less than about 3%, less than about 2%, less than about 1%, or preferably, no taper, so that the diameter actually is constant. Taper may be measured as diameter increase over length of the taper. By way of example, a 10% taper (e.g., 10% increase in centimeters per 1 cm) across a 2 cm tip portion, where the extreme distal end of the distal tip had a diameter of 0.0022 inch, may provide the extreme proximal end of the proximal tip portion with a diameter of 0.00264 inch. Such shallow tapers may be sufficiently insignificant to ensure that the torsional deformation is preferentially present within the superelastic proximal tip portion.

Advantageously, tip portion 218 includes both a section (portion 222) that exhibits superelastic characteristics, which yields more readily and typically endures greater torsional deformation than the distal portion 224, which has been cold worked to remove its superelastic characteristics, rendering the material of distal portion 224 linear elastic. Further proximal core wire 202 (tapered in Figure 15B) may also be superelastic nitinol, and may similarly be formed of a single integral piece of material with proximal tip portion 222. Because tip portion 218 includes both a proximal superelastic portion and a distal linear elastic portion, the guide wire has been found to exhibit greater durability in terms of turns to failure than if the small and short substantially constant diameter tip 218 were comprised entirely of the more shapeable previously cold worked material. Such increased durability is particularly advantageous during use, where failure of a guide wire distal portion within a patient's vasculature is very undesirable.

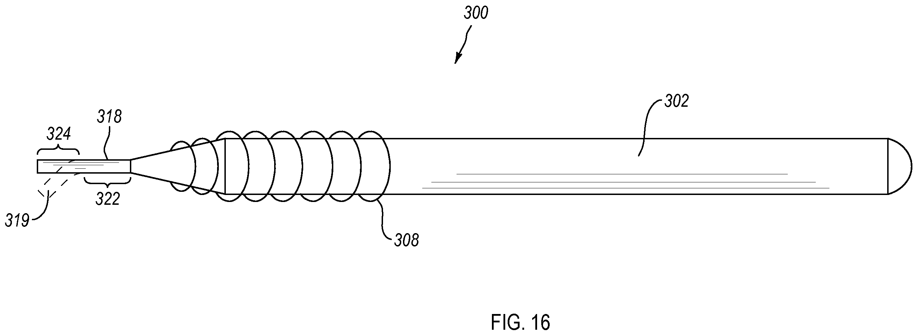

Figure 16 shows a simplified embodiment of another intravascular guide wire 300 including features of the present disclosure. Guide wire 300 is shown as including a core wire 302, with a coil 308 disposed over a part of core wire 302. Similar to as described above, rather than flattening distal tip 318 so as to render it more easily permanently deformable, which results in a rectangular cross-section to flattened tip 318, tip 318 may be provided with portions 322 and 324 which are both circular in cross-section, and of substantially the same diameter, but with different properties. Proximal tip portion 322 may exhibit superelastic properties, while distal tip portion 324 may be cold worked to exhibit linear elastic properties, rather than superelastic properties. Because of its linear elastic properties, distal tip portion 324 is more easily bent as shown by bend 319.

For example, superelastic nitinol may exhibit an elastic strain limit of about 8%, which is remarkably higher than for many other metal materials, and is thus referred to as super-elastic. By way of comparison, spring temper 300 series stainless steels may exhibit an elastic strain limit of about 1%. The very high elastic strain limit of such superelastic materials is beneficial when attempting to navigate through tortuous vasculature, but makes it difficult to impart a permanent bend to a wire of such a material. As described herein, often a practitioner will wish to impart a J-bend, L-bend, or other bend into the extreme distal tip of the guide wire core prior to clinical use. By imparting cold work to the distal tip portion 224, 324, this portion can be made to exhibit linear elastic, rather than superelastic characteristics as the initially austenitic structure is transformed to martensite, through application of the cold work. Such linear elastic nitinol may exhibit an elastic strain limit of only about 2% to about 4%, significantly lower than in its superelastic state, making it much easier for a practitioner to impart a permanent bend to this tip portion. By not cold working the entire tip portion 218, 318, but ensuring that the tip portion 218, 318 includes both a proximal superelastic portion and a distal linear elastic portion, both having the same cross-sectional circular shape and substantially same diameter, improved durability is provided in terms of turns to failure, as described in the comparative examples included herein. For example, the resulting tip portion can accept more torsional deformation before failure than would be possible if the entire tip were formed from the linear elastic material, as shown by the comparative testing results included herein.

The illustrated configurations for guide wires 200, 300 are merely two of many possible configurations, and other guide wire configurations including a tip portion of circular cross-section and substantially constant diameter, including both a superelastic proximal tip portion and a linear elastic distal tip portion are encompassed by the present disclosure.

Any suitable superelastic material may be employed for the tip portion, prior to cold working the distal tip portion thereof so as to render it linear elastic. Nitinol (a nickel-titanium alloy), or another superelastic alloy may be employed. In an embodiment, a suitable nitinol alloy may include about 30 atomic percent to about 52 atomic percent titanium, with the balance typically being nickel. Optionally, a small amount of other alloying elements may be included. For example, up to about 10 atomic percent or up to about 3 atomic percent of iron, cobalt, vanadium, platinum, palladium, copper, and combinations thereof may be added, if desired.

Addition of nickel above equiatomic amounts relative to titanium increases stress levels at which the stress induced austenite to martensite transition occurs. This characteristic can be used to ensure that the temperature at which the martensitic phase thermally transforms to the austenitic phase is well below human body temperature (37°C). Of course, as described above, the martensitic phase may be cold work induced within the distal tip portion. Excess nickel may also provide an expanded strain range at very high stresses when the stress induced transition occurs during use.

Because of the extended strain range characteristics of nitinol, a guide wire made of such material can be more readily advanced through tortuous arterial passageways with minimal risk of kinking, as compared to say, stainless steel. Such characteristics are similarly beneficial where the guide wire may be prolapsed, either deliberately or inadvertently.

While the distal tip of the guide wire may comprise an alloy capable of exhibiting superelastic properties (although the superelastic properties may be eliminated through cold working), it will be appreciated that more proximal portions of the guide wire may be formed of a material exhibiting greater strength and less flexibility than the selected superelastic material. For example, more proximal portions of the guide wire may be formed of stainless steel, cobalt-chromium alloys such as MP35N, or other materials exhibiting greater strength (e.g., higher tensile strength) than the superelastic capable material of the tip portion.

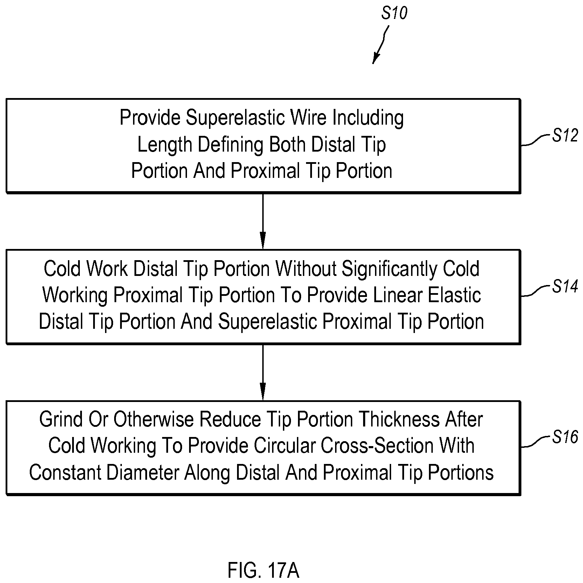

Figure 17A illustrates an exemplary method S10, by which a tip portion of a guide wire may be formed. At S12, a superelastic wire including a length defining both a distal tip portion and a proximal tip portion is provided. Such a wire may be significantly longer than just the tip portion 218 or 318 of guide wires 200 and 300 seen in Figures 15A and 16. For example, all or a portion of core wire 202 or 302 (e.g., any portion thereof that is also formed of nitinol or other superelastic alloy) may also be formed from this same provided superelastic wire. For example, the superelastic wire may include a length that defines both the distal and proximal tip portions, as well as at least a portion of the remainder of core wire 202, or 302.

The length of that portion of the core wire 202 or 302 including tapered sections (e.g., 206 of Figure 15A) may be about 10 cm to about 40 cm in length, about 2 cm to about 6 cm in length. In an embodiment, the substantially constant diameter distal tip 218 or 318 may be about 2 cm in length. Where some of the portion of core wire 202 or 302 may also be formed of the superelastic wire, the length of the provided wire in step S12 may be significantly longer. For example, the proximal core section 202 of the guide wire device 200 may generally be about 130 cm to about 280 cm in length with an outer diameter of about 0.006 inch to 0.018 inch (0.15 mm - 0.45 mm), or about 0.010 inch to about 0.015 inch (0.25 mm - 0.38 mm) for coronary use. Larger diameter guide wires, e.g. up to 0.035 inch (0.89 mm) or more may be employed in peripheral arteries and other body lumens. As described above, the length of the more distal smaller diameter and tapered sections can range from about 10 cm to about 40 cm, depending upon the particular guide wire. The helical coiled section 208 may be about 3 cm to about 45 cm in length, e.g., about 5 cm to about 20 cm. In any case, it will be apparent that the wire provided in step S12 may have a length greater than just that of the tip portion 218, as it may provide some or all of the proximally disposed sections of core wire 102 as well. In addition, a small length (e.g., 5 mm) at the distal end of the wire may be trimmed therefrom, e.g., after final grinding.

At S14, the distal tip portion of the tip is cold worked, without imparting significant cold work to the proximal tip portion. This provides linear elastic properties within the distal tip portion, while providing superelastic properties within the proximal tip portion. After application of the cold work, at S16, the tip portion (e.g., proximal tip portion, the distal tip portion, or both) is ground or otherwise reduced in size (e.g., cross-sectional thickness) to provide a circular cross-section with a substantially constant diameter along the distal and proximal tip portions.

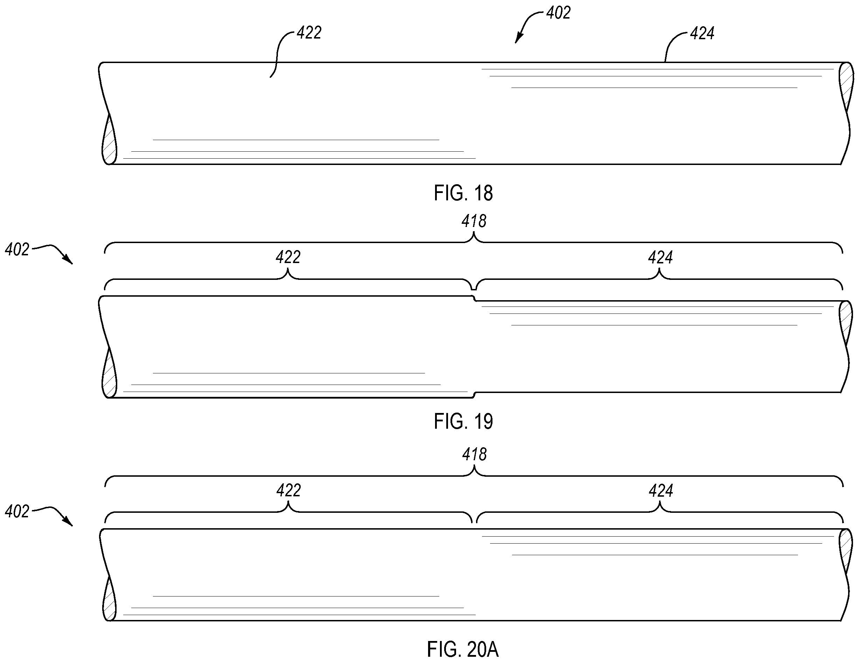

Figures 18-22 progressively illustrate such an exemplary method. For example, as shown in Figure 18, a wire 402 (e.g., formed of a material capable of exhibiting superelatic properties, such as nitinol) is provided. Wire 402 may be prepared by any suitable method. For example, such wire is commercially available, and may have been formed from an ingot which itself may have been formed by melting and casting using a vacuum induction or vacuum arc melting process. Such an ingot may then have been forged, rolled, and drawn into a wire. In any case, wire 402, whether provided already formed or formed from an ingot or other starting material is sufficiently long so as to define at least both a distal tip portion 424 and a proximal tip portion 422. Commercially available wire may be drawn down from an as provided diameter, to a smaller diameter closer to the final diameter of the desired guide wire. After any such initial drawing, the wire may be heat treated to restore superelasticity (e.g., partial anneal at about 500°C for 3 to 10 minutes).

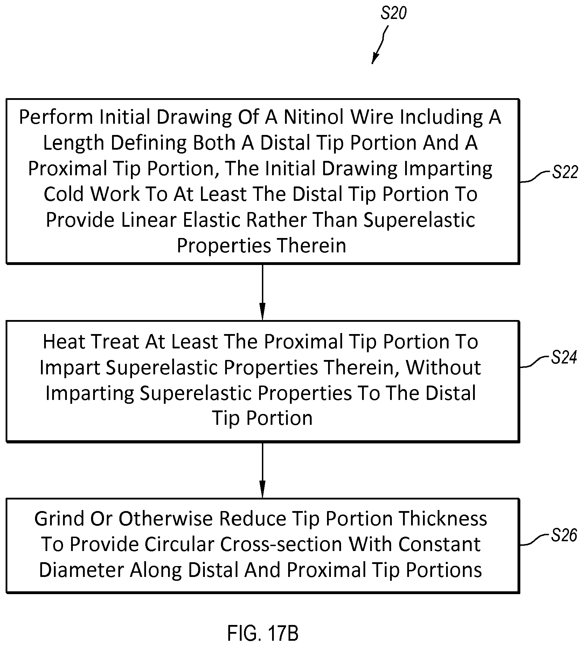

Another embodiment of a suitable method (S20) is shown in Figure 17B, and may include initially drawing the wire as described above (S22), but rather than heat treating the wire to impart superelasticity throughout its full length, only a portion of the as-drawn wire is heat treated to impart superelasticity, imparting super-elasticity everywhere except the distal tip portion, where linear elastic properties are desired (S24). Such a wire may initially be in a superelastic condition, or may have been fully annealed prior to drawing. After grinding or other cross-section reduction process (S26), the result is similar - providing of a linear elastic distal tip portion 424 and a superelastic proximal tip portion 422. Nitinol or any other superelastic capable alloy material may be used in any of the methods described herein. As such, the term "nitinol" as used herein is to be broadly construed, to include other superelastic capable materials as well.

Returning to description of an embodiment where superelastic properties have been imparted after initial drawing, in its as-provided condition as referred to in Figure 17A, wire 402 may exhibit superelastic characteristics across both portions 422 and 424. As described herein, it is desirable that distal tip portion 424 be altered so as to exhibit linear elastic, rather than superelastic properties, but while ensuring that the finished tip portion of the guide wire include a circular cross-section, of substantially constant diameter across both portions 422 and 424.

As seen in Figure 19, cold work may be applied to distal tip portion 424, e.g., by rotary swaging, wire drawing, or another cold working mechanism (e.g., tensioning, rolling, stamping, coining, etc.). Advantageously, such cold working may be imparted prior to performing any final grinding of wire 402 or otherwise reducing the thickness (e.g., diameter) of wire 402. A sufficient amount of cold work may be applied to distal tip portion 424 so as to ensure that tip portion 424 exhibits linear elastic, rather than superelastic properties.

Preferably such cold work is imparted by rotary swaging, rather than wire drawing, as wire drawing typically imparts a curl to the wire as it is drawn, which curl may be removed by subsequent mechanical straightening and heat treatment of the curled wire (e.g., by simultaneously applying heat and tension to the curled wire). Such heat treatments are low enough in temperature and/or time to not restore the original superelastic properties of the wire (e.g., which may take several minutes exposure at about 500°C).

Rotary swaging does not impart any significant curl to the cold worked wire, so long as the axial feed is aligned with the swaging mechanism. Rotary swaging may involve use of a set of two or more revolving dies which radially deform the wire as it passes between the dies. Rotary swaging also advantageously may not significantly alter the original circular cross-section shape of the wire (other than making it somewhat smaller), thus maintaining the original and desired circular cross-sectional geometry. In an embodiment, the closed dies may provide a football shaped lumen, and may execute multiple openings and closures per revolution. The swaging dies may operate at from about 500 RPM to about 1000 RPM, from about 600 RPM to about 900 RPM, or from about 750 RPM to about 850 RPM (e.g., 800 RPM). Commercially available swaging machines may be employed, e.g., as available from Torrington Machinery, Waterbury, CT.

Rotary swaging can result in so-called redundant work, caused by repeated blows to a given location of the wire as the dies and wire are rotated relative to one another. As a result, the measured percentage area reduction of the wire may indicate less cold work than is actually incurred by the wire material (as a result of greater redundant cold work than in more conventional wire deformation processes such as drawing, rolling, or stamping). The amount of redundant cold work, can be affected by axial feed rate, the die strike rate, and the geometry of the dies (e.g., contact length and contact surface area and surface shape provided by the die), ratio of die contact surface length to wire diameter, and other factors. Further, the distribution of redundant cold work can vary throughout the cross-section of the wire, with typically higher redundant cold work occurring near the center. As such, it can be important to carefully select appropriate processing conditions when imparting the desired cold work by rotary swaging before grinding or otherwise reducing wire thickness of the tip portion of the guide wire core wire.

In any case, the amount of cold work imparted to the distal tip portion 424 is sufficient so that the distal tip portion exhibits linear elastic, rather than superelastic characteristics. For example, it may exhibit an elastic strain limit of less than 6%, less than 5%, no more than about 4%, or from about 2% to about 4% after cold working, rather than the approximately 8% elastic strain limit that may be exhibited by the proximal tip portion 422.

As seen in Figure 19, as a result of rotary swaging or other cold working, the diameter of distal tip portion 424 may be somewhat reduced relative to its initial diameter, and the diameter of proximal tip portion 422, which was not subjected to any significant cold work. The reduction in diameter may be no more than 15%, no more than 10%, from about 5% to about 15%, or 5% to about 10%, depending on the mode by which cold work was applied, and the amount of cold work applied. Similarly, the reduction in cross-sectional area of distal tip portion 424 may be from about 15% to about 25%, or about 15% to about 20%. The amount of cold work may be from about 20% to about 30%, which may be somewhat higher than the reduction in cross-sectional area due to redundant cold work. More generally, the amount of cold work may be from about 15% to about 50%, from about 15% to about 40%, or from about 20% to about 30%. In any case, the amount of cold work applied may be sufficient to render the nitinol or other initially superelastic material of distal tip portion 424 linear elastic, rather than superelastic. Proximal tip portion 422 may advantageously continue to exhibit superelastic characteristics.

As seen in Figure 20A, after cold working, at least a portion of tip portion 418 of wire 402 (e.g., at least proximal tip portion 422) may be ground or otherwise reduced in cross-section, so as to provide a circular cross-section of substantially the same diameter along both portions 422 and 424. While in theory grinding may be possible before cold working, the finished diameter of portion 418 after grinding is so small as to make this difficult, if not impossible as a practical matter. For this reason, the cold work may be applied before grinding or other reduction in cross-section.

In an embodiment, grinding or other removal may be limited to proximal tip section 422, while in another embodiment, (e.g., see Figure 20B), thickness may be removed from both proximal and distal tip portions 422 and 424. It may be preferred to remove thickness from both portions 422 and 424 to remove any dimpled surface, or minor alteration of the cross-section of distal portion 424 that may result from cold working. For example, the rotary swaging operation where the surface of portion 424 is subjected to radial blows from opposed dies may in some circumstances result in a somewhat dimpled surface, at least on a microscopic level, depending upon the number of die strikes per location (e.g., with more die strikes generally producing smoother surfaces). Final grinding to a desired final diameter across both portions 422 and 424 ensures that any such modification of the surface, or alternation of the cross-section of distal tip portion 424 is removed, providing a circular cross section with a smooth outer surface. Similarly, tapering present in any part of wire 402 that is proximal to distal tip 418 may be introduced at this stage

It will be appreciated that the initial wire may have an extreme distal portion thereof trimmed off (e.g., about 3 mm to 10 mm, or 4 mm to 6 mm) after cold working, e.g., after final grinding, to provide the linear elastic distal tip portion of the desired final length. Distal trimming serves to eliminate abnormalities in surface finish or dimension which may sometimes result from the final grinding process.

By way of example, the grinding or other process for reducing the cross-sectional thickness of tip portion 418 may be a centerless grinding operation, which is a machining process that employs abrasive grinding to remove material from the tip portion 418. In some embodiments, the tip portion 418 may be held between a workholding platform and two wheels rotating in the same direction, at different speeds. One wheel, referred to as the regulating wheel, may be on a fixed axis, and may rotate such that the force applied to tip portion 418 is directed downward, against the work holding platform. The regulating wheel may impart rotation to the tip portion 418 by its having a higher speed than the other wheel. The other wheel, referred to as the grinding wheel, is movable. The grinding wheel may be positioned to apply lateral pressure to the tip portion 418, and may include a rougher or rubber-bonded adhesive to grind away material from the tip portion 418. The speed of the two wheels relative to one another provides the rotating action to tip portion 418, and may determine the rate at which material is removed from the tip portion 418 by the grinding wheel. For example, during operation the tip portion 418 may turn with the regulating wheel, with the same linear velocity at the point of contact. The grinding wheel may turn faster, slipping past the surface of the tip portion 418 at the point of contact, removing material as it passes. Although centerless grinding may be preferred for removing material thickness from the tip portion 418 after cold working, so as to provide the desired circular cross-section having a substantially constant diameter across both portions 422 and 424 of tip portion 418, it will be appreciated that other removal techniques may be employed (e.g., chemical etching, electrochemical polishing, etc.)

As seen in Figure 21, if desired, a coating or other exterior jacket or layer 426 may be applied over at least a portion of core wire 402 and/or tip portion 418. Such a layer 426 may include a lubricious polymer with hydrophilic, or even hydrophobic properties, as desired.

Figure 22 illustrates a tip portion 518 of core wire 502 in which the proximal tip portion 522 and the distal tip portion 524 are approximately equal in length. Because tip portion 518 may be the extreme distal end of the guide wire core of a guide wire device, as shown in Figures 15A-16, the inventors have found it to be particularly advantageous that the tip portion 518, which includes a substantially constant diameter along its entire length, include both a distal linear elastic portion 524 (which can advantageously be bent by the practitioner, while maintaining the desired circular cross section), and a proximal superelastic portion 522.

Furthermore, the inventors have found that it is particularly advantageous to provide a tip portion where the linear elastic distal tip portion has a length that is about 30% to about 70% that of a combined length of the distal tip portion 524 and the superelastic proximal tip portion 522. As a result, the superelastic proximal tip portion may also have a length that also is about 30% to about 70% that of the combined length. In an embodiment, the distal tip portion has a length that is about 50% that of the combined length, so that the lengths of the distal and proximal tip portions are approximately equal to one another. Stated another way, the proximal tip portion length may be from about 40% to about 230%, from about 50% to about 200%, from about 75% to about 150%, or from about 75% to about 125% that of the distal tip portion length. Likewise, the distal tip portion length may be from about 40% to about 230%, from about 50% to about 200%, from about 75% to about 150%, or from about 75% to about 125% that of the proximal tip portion length. Examples of various formed and tested tip portions, including their proximal tip portion lengths relative to the distal tip portion length are shown in Table 1B, below.

In an embodiment, the combined length of the proximal and distal tip portions may be from about 1 cm to about 6 cm, from about 1 cm to about 4 cm, from about 1.5 cm to about 3 cm, or from about 1.5 cm to about 2.5 cm (e.g., about 2 cm). The length of the superelastic proximal tip portion may be from about 0.3 cm to about 3 cm, from about 0.5 cm to about 2 cm, or from about 0.75 cm to about 1.25 cm (e.g., about 1 cm in length). The length of the linear elastic distal tip portion may be from about 0.3 cm to about 3 cm, from about 0.5 cm to about 2 cm, or from about 0.75 cm to about 1.25 cm (e.g., about 1 cm in length).

Comparative testing was conducted using various tip portion configurations as described below, illustrating the benefits of providing both proximal superelastic and distal linear elastic portions in the tip. The results in Table 1A show particularly improved results for such relative length fractions as described above - e.g., where equal lengths of super elastic and linear elastic proximal and distal tip portions are provided, in terms of greater durability in turns to failure (TTF) results. Table 1B quantifies the proximal tip portion length relative to the distal tip portion length for examples 1-8.

**Table 1A**

| Example | Distal Tip Portion Length (mm) | Proximal Tip Portion Length (mm) | Combined Tip Length (mm) | Dia. (inch) | TTF (avg.) | TTF (Std. Dev.) |
|---|---|---|---|---|---|---|
| 1 | 10 | 5 | 15 | 0.0022 | 19.50 | 1.84 |
| 2 | 10 | 5 | 15 | 0.0024 | 17.80 | 2.15 |
| 3 | 10 | 10 | 20 | 0.0022 | 22.33 | 1.51 |
| 4 | 10 | 10 | 20 | 0.0024 | 22.80 | 1.79 |
| 5 | 15 | 0 | 15 | 0.0022 | 16.55 | 1.04 |
| 6 | 15 | 0 | 15 | 0.0024 | 14.71 | 2.69 |
| 7 | 15 | 5 | 20 | 0.0022 | 19.00 | 2.26 |
| 8 | 15 | 5 | 20 | 0.0024 | 20.80 | 1.69 |

**Table 1B**

| Example | Distal Tip Portion Length (mm) | Proximal Tip Portion Length (mm) | Proximal Tip Length Relative to Distal Tip Length |
|---|---|---|---|
| 1 | 10 | 5 | 50% |
| 2 | 10 | 5 | 50% |
| 3 | 10 | 10 | 100% |
| 4 | 10 | 10 | 100% |
| 5 | 15 | 0 | 0% |
| 6 | 15 | 0 | 0% |
| 7 | 15 | 5 | 33% |
| 8 | 15 | 5 | 33% |

Ten samples of each of examples 1, 2, 7, and 8 were tested, while 5 samples of example 4, 6 samples of example 3, 7 samples of example 7, and 11 samples of example 5 were tested. Each sample was prepared in the same way, including removal of a 5 mm distal section from the wire after rotary swaging. The reported distal tip portion lengths are final lengths, after trimming off a 5 mm section. Examples 3 and 4 exhibited the highest TTF results. These examples included 10 mm linear elastic distal tip portion lengths, 10 mm superelastic proximal tip portion lengths, and 20 mm combined tip lengths. Examples 5 and 6 exhibited the lowest TTF results, and included a 15 mm linear elastic distal tip portion length, and no superelastic proximal tip portion (i.e., the entire substantially constant diameter tip portion was linear elastic). The other examples exhibited TTF results between these two extremes. For example, examples 3 and 7, whose factors match except for the distal tip portion length (10 mm versus only 5 mm), differ by an average of more than 3 turns, while examples 1 and 5 also differ on average by nearly 3 turns.

In TTF testing, a proximal end of the guide wire is rotated while fixing the distal tip of the guide wire. Deformation tended to occur within the distal tip, as it represents the smallest cross-section within the guide wire. Deformation tended to localize at any appropriate interface or change in cross-section (e.g., where the taper begins, etc.). In examples including a superelastic proximal tip portion, the deformation tended to concentrate within the superelastic portion, which was advantageous, as this portion is more flexible and more durable due to its greater ductility.

In some embodiments, the tip portion of the guide wire may exhibit at least 18 turns to failure on average, at least 20 turns to failure on average, at least 21 turns to failure on average, or at least 22 turns to failure on average.

Table 2 below shows the percentage increase in durability as measured by TTF for each example, as compared to the corresponding control examples 5 and 6, having the same diameter (i.e., examples 1, 3, and 7 are compared to example 5, as they all have the same diameter, and examples 2, 4, and 8 are compared to example 6, as they all have the same diameter).

**Table 2**

| Example | Distal Tip Portion Length (mm) | Proximal Tip Portion Length (mm) | Combined Tip Length (mm) | Dia. (inch) | TTF (avg.) | Change Relative to Control |
|---|---|---|---|---|---|---|
| 1 | 10 | 5 | 15 | 0.0022 | 19.50 | +18% |
| 2 | 10 | 5 | 15 | 0.0024 | 17.80 | +21% |
| 3 | 10 | 10 | 20 | 0.0022 | 22.33 | +35% |
| 4 | 10 | 10 | 20 | 0.0024 | 22.80 | +55% |
| 5 | 15 | 0 | 15 | 0.0022 | 16.55 | - |
| 6 | 15 | 0 | 15 | 0.0024 | 14.71 | - |
| 7 | 15 | 5 | 20 | 0.0022 | 19.00 | +15% |
| 8 | 15 | 5 | 20 | 0.0024 | 20.80 | +41% |

For example, the increase in average turns to failure as compared to an otherwise identical tip portion where the entire distal tip portion having a circular cross-sectional and substantially constant diameter were linear elastic, may be at least about 15%, at least about 20%, at least about 25%, at least about 30%, from about 15% to about 60%, from about 15% to about 55%, from about 20% to about 55%, from about 25% to about 55%, or from about 30% to about 55%. Such percentage increases are significant, as the guide wires are often employed in environments where the vasculature or other pathway to be followed can be quite tortuous. Failure of a guide wire within a patient, during a procedure is particularly undesirable. Thus, the presently described guide wires and methods of manufacture reduce risk of such failure, while at the same time providing for improved torque response due to the presence of a distal tip of circular cross-section and substantially constant diameter.

Some embodiments of the invention may include a multi-piece distal tip construction. For example, where a relatively more shapable distal tip portion may be bonded to a more durable superelastic segment (e.g., by butt welding, or other suitable joinder method). For multi-piece distal tip constructions, the more shapable tip portion may comprise cold worked nitinol, a different composition of nitinol than the proximal tip portion, or a material other than nitinol, such as stainless steel, MP35N, or other cobalt-chromium alloy. Any other features of the multi-piece distal tip may be as described herein (e.g., circular cross-section, substantially constant diameter, lengths disclosed above, etc.).

The present invention may be embodied in other specific forms without departing from its essential characteristics. The described embodiments are to be considered in all respects only as illustrative and not restrictive. The scope of the invention is, therefore, indicated by the appended claims rather than by the foregoing description.

## Claims

1. A method for manufacturing a tip portion (218, 318) of a guide wire (200, 300), the method comprising:
providing a superelastic wire (202, 302) including a length so as to define both a distal tip portion (224, 324) and a proximal tip portion (222, 322);
cold working the distal tip portion (224, 324) without imparting significant cold work to the proximal tip portion (222, 322) to provide linear elastic rather than superelastic properties within the distal tip portion (224, 324), so that the proximal tip portion (222, 322) exhibits superelastic properties and the distal tip portion (224, 324) exhibits linear elastic properties; and
grinding or otherwise reducing a cross-sectional thickness of the tip portion (218, 318) after cold working to provide a circular cross-section having a desired substantially constant diameter along the distal tip portion and proximal tip portion (222, 322) of the tip.

2. The method of claim 1, wherein cold working the distal tip portion (224, 324) comprises rotary swaging the distal tip portion (224, 324) without imparting significant cold work to the proximal tip portion (222, 322); preferably wherein rotary swaging reduces a diameter of the distal tip portion (224, 324) by no more than about 15%, or wherein rotary swaging reduces a diameter of the distal tip portion (224, 324) by about 5% to about 15%.

3. A method for manufacturing a tip portion (218, 318) of a guide wire (200, 300), the method comprising:
providing a superelastic wire (202, 302) including a length so as to define both a distal tip portion (224, 324) and a proximal tip portion (222, 322);
rotary swaging the distal tip portion (224, 324) without imparting significant cold work to the proximal tip portion (222, 322) to provide linear elastic rather than superelastic properties within the distal tip portion (224, 324), so that the proximal tip portion (222, 322) exhibits superelastic properties and the distal tip portion (224, 324) exhibits linear elastic properties; and
grinding the tip portion (218, 318) after cold working to provide a circular cross-section having a desired substantially constant diameter along the distal tip portion (224, 324) and proximal tip portion (222, 322) of the tip;
wherein the distal tip portion (224, 324) has a finished length that is about 30% to about 70% that of a combined finished length of the distal tip portion (224, 324) and the proximal tip portion (222, 322).

4. The method of claim 3, wherein rotary swaging reduces a diameter of the distal tip portion (224, 324) by no more than about 15%; wherein rotary swaging reduces a diameter of the distal tip portion (224, 324) by about 5% to about 15%; or wherein the distal tip portion (224, 324) has a finished length that is about 50% that of a combined finished length of the distal tip portion (224, 324) and the proximal tip portion (222, 322); or wherein the combined finished length of the distal tip portion (224, 324) and the proximal tip portion (222, 322) is about 2 cm, the proximal tip portion (222, 322) having a finished length of about 1 cm and the distal tip portion (224, 324) having a finished length of about 1 cm.

5. A method for manufacturing a tip portion (218, 318) of a guide wire (200, 300), the method comprising:
performing initial drawing of a nitinol wire, the wire having a length so as to define both a distal tip portion (224, 324) and a proximal tip portion (222, 322) of the guide wire (200, 300), the initial drawing imparting sufficient cold work to at least the distal tip portion (224, 324) to provide linear elastic rather than superelastic properties within the distal tip portion (224, 324);
heat treating at least the proximal tip portion (222, 322) of the wire to ensure superelastic properties arc provided within the proximal tip portion (222, 322), without imparting superelastic properties to the distal tip portion (224, 324) so that the proximal tip portion (222, 322) exhibits superelastic properties and the distal tip portion (224, 324) exhibits linear clastic properties;
**characterized in that**, that the method further comprises grinding or otherwise reducing a cross-sectional thickness of the tip portion (218, 318) to provide a circular cross-section having a desired substantially constant diameter along the distal tip portion (224, 324) and proximal tip portion (222, 322) of the tip.

6. The method of claim 5, wherein the initial drawing renders both the proximal (222, 322) and distal (224, 324) tip portions linear elastic, the heat treating of the proximal tip portion (222, 322) imparting superelastic properties to the proximal tip portion (222, 322) without imparting superelastic properties to the distal tip portion (224, 324); or wherein the distal tip portion (224, 324) has a finished length that is about 50% that of a combined finished length of the distal tip portion (224, 324) and the proximal tip portion (222, 322); or wherein the combined finished length of the distal tip portion (224, 324) and the proximal tip portion (222, 322) is about 2 cm, the proximal tip portion (222, 322) having a finished length of about 1 cm and the distal tip portion (224, 324) having a finished length of about 1 cm.

7. A guide wire (200, 300) comprising:
a guide wire tip portion (218, 318) including a distal tip portion (224, 324) and a proximal tip portion (222, 322), the tip portion (218, 318) including a substantially constant diameter along both the distal tip portion (224, 324) and the proximal tip portion (222, 322);
the distal tip portion (224, 324) having a circular cross-section and exhibiting linear elastic rather than superelastic properties; and
the proximal tip portion (222, 322) having a circular cross-section and exhibiting superelastic properties;
**characterized in that** the distal tip portion (224, 324) and the proximal tip portion (222, 322) are integrally formed from a single piece of material so as to not include any joint therebetween.

8. The guide wire (200, 300) of claim 7, wherein the distal tip portion (224, 324) has a length that is about 30% to about 70% that of a combined length of the distal tip portion (224, 324) and the proximal tip portion (222, 322); or wherein the distal tip portion (224, 324) has a length that is about 50% that of a combined length of the distal tip portion (224, 324) and the proximal tip portion (222, 322); or wherein a combined length of the distal tip portion (224, 324) and the proximal tip portion (222, 322) is about 2 cm, the proximal tip portion (222, 322) having a length of about 1 cm and the distal tip portion (224, 324) having a length of about 1 cm.

9. The guide wire (200, 300) of claim 7, wherein the tip portion (218, 318) exhibits at least 18 turns to failure on average; or wherein the tip portion (218, 318) exhibits at least 20 turns to failure on average; or wherein the tip portion (218, 318) exhibits at least 22 turns to failure on average; or wherein the tip portion (218, 318) exhibits at least a 15% increase in average turns to failure as compared to an otherwise identical tip portion (218, 318) where the entire distal tip (224, 324) portion having a circular cross-sectional and substantially constant diameter were linear elastic; or wherein the tip portion (218, 318) exhibits at least a 30% increase in average turns to failure as compared to an otherwise identical tip portion (218, 318) where the entire distal tip portion (224, 324) having a circular cross-sectional and substantially constant diameter were linear elastic.

10. A method of making a cure metal element for a medical guide wire (200, 300) comprising:
providing a wire (112a) of nickel titanium alloy having a length that includes a proximal portion (116) having a first diameter and a distal portion having a second diameter;
applying cold work to the distal portion (118) and not applying cold work to the proximal portion (116), thereby imparting to the distal portion (118) a third diameter that is smaller than the second diameter; and then
applying a reducing process to the wire whereby the proximal portion (116) is reduced to have a fourth diameter that is less than the first diameter;
**characterized in that** the reducing process results in a circular cross-section along the distal portion (118) and proximal portion (116) that is substantially constant in diameter.

11. The method of claim 10, wherein providing a wire (112a) includes providing a wire (112a) with superelastic properties throughout the length; or wherein providing a wire (112a) includes providing a wire (112a) wherein the proximal portion (116) is adjacent the distal portion (118); or wherein providing a wire (112a) includes providing a wire (112a) wherein the proximal portion (116) is adjacent a proximal end of the wire (112a); or wherein providing a wire (112a) includes providing a wire (112a) wherein the distal portion (118) is adjacent a distal end of the wire (112a).

12. The method of claim 10, wherein applying cold work to the distal portion (118) includes applying sufficient cold work to render the distal portion (118) to have linear elastic properties, preferably wherein after applying cold work to the distal portion (118), the proximal portion (116) retains superelastic properties, more preferably wherein no joint is inserted into the wire (112a) over the length; or more preferably wherein no welding process is applied to the wire (112a) over the length, most preferably further including removing the guide wire (200, 300) from the die without drawing the distal portion (1 18) back through the die.

13. The method of claim 10, wherein applying a reducing process to the guide wire (200, 300) includes applying centerless grinding; or wherein providing a wire (112a) includes providing a wire (112a) wherein the first diameter is the same as the second diameter; or wherein applying a reducing process to the wire (112a) includes reducing the proximal portion (116) to have a fourth diameter that is the same as the third diameter; or wherein applying a reducing process to the wire (112a) includes reducing the distal portion (118) to have a fifth diameter that is less than the third diameter; or wherein applying cold work to the distal portion (118) includes drawing the distal portion (118) through a die, preferably wherein applying cold work to the distal portion (118) includes applying cold work methods selected from: swaging, rolling, tensioning, stamping, coining, and combinations thereof.

14. A medical guide wire (200, 300) comprising:
a solid metal core (112) having a length and having a constant diameter over the length, wherein the length includes a proximal portion (116) having pseudoelastic properties and a distal portion (118) having linear elastic properties, and wherein the length of the core (112) does not include a mechanical joint at any location between the proximal portion (116) and the distal portion (118).

15. The guide wire of claim 14, wherein the proximal portion (116) is formed from a nickel titanium alloy; or wherein the distal portion (118) includes metal to which the linear elastic properties have been imparted by a process of cold working.

## Patentansprüche

1. Verfahren zum Herstellen eines Spitzenabschnitts (218, 318) eines Führungsdrahtes (200, 300), welches Verfahren umfasst:
Bereitstellen eines superelastischen Drahtes (202, 302) umfassend eine Länge, um sowohl einen distalen Spitzenabschnitt (224, 324) als auch einen proximalen Spitzenabschnitt (222, 322) zu definieren;
Kaltverarbeitung des distalen Spitzenabschnitts (224, 324), ohne dem proximalen Spitzenabschnitt (222, 322) eine signifikante Kaltverarbeitung zu verleihen, um eher lineare elastische als superelastische Eigenschaften innerhalb des distalen Spitzenabschnitts (224, 324) bereitzustellen, so dass der proximale Spitzenabschnitt (222, 322) superelastische Eigenschaften aufweist, und der distale Spitzenabschnitt (224, 324) lineare elastische Eigenschaften aufweist; und
Schleifen oder anderweitiges Reduzieren einer Querschnittsdicke des Spitzenabschnitts (218, 318) nach der Kaltverarbeitung, um einen kreisförmigen Querschnitt bereitzustellen, der einen gewünschten im Wesentlichen konstanten Durchmesser entlang des distalen Spitzenabschnitts und proximalen Spitzenabschnitts (222, 322) der Spitze hat.

2. Verfahren nach Anspruch 1, wobei die Kaltverarbeitung des distalen Spitzenabschnitts (224, 324) das Rundkneten des distalen Spitzenabschnitts (224, 324) umfasst, ohne dem proximalen Spitzenabschnitt (222, 322) eine signifikante Kaltverarbeitung zu verleihen; bevorzugt wobei das Rundkneten einen Durchmesser des distalen Spitzenabschnitts (224, 324) um nicht mehr als ungefähr 15 % reduziert, oder wobei das Rundkneten einen Durchmesser des distalen Spitzenabschnitts (224, 324) um ungefähr 5 % bis ungefähr 15 % reduziert.

3. Verfahren zum Herstellen eines Spitzenabschnitts (218, 318) eines Führungsdrahtes (200, 300), welches Verfahren umfasst:
Bereitstellen eines superelastischen Drahtes (202, 302) umfassend eine Länge, um sowohl einen distalen Spitzenabschnitt (224, 324) als auch einen proximalen Spitzenabschnitt (222, 322) zu definieren;
Rundkneten des distalen Spitzenabschnitts (224, 324), ohne dem proximalen Spitzenabschnitt (222, 322) eine signifikante Kaltverarbeitung zu verleihen, um eher lineare elastische als superelastische Eigenschaften innerhalb des distalen Spitzenabschnitts (224, 324) bereitzustellen, so dass der proximale Spitzenabschnitt (222, 322) superelastische Eigenschaften aufweist, und der distale Spitzenabschnitt (224, 324) lineare elastische Eigenschaften aufweist; und
Schleifen des Spitzenabschnitts (218, 318) nach der Kaltverarbeitung, um einen kreisförmigen Querschnitt bereitzustellen, der einen gewünschten im Wesentlichen konstanten Durchmesser entlang des distalen Spitzenabschnitts (224, 324) und proximalen Spitzenabschnitts (222, 322) der Spitze hat;
wobei der distale Spitzenabschnitt (224, 324) eine Fertiglänge hat, die ungefähr 30 % bis ungefähr 70 % einer kombinierten Fertiglänge des distalen Spitzenabschnitts (224, 324) und des proximalen Spitzenabschnitts (222, 322) beträgt.

4. Verfahren nach Anspruch 3, wobei das Rundkneten einen Durchmesser des distalen Spitzenabschnitts (224, 324) um nicht mehr als ungefähr 15 % reduziert; wobei das Rundkneten einen Durchmesser des distalen Spitzenabschnitts (224, 324) um ungefähr 5 % bis ungefähr 15 % reduziert; oder wobei der distale Spitzenabschnitt (224, 324) eine Fertiglänge hat, die ungefähr 50 % einer kombinierten Fertiglänge des distalen Spitzenabschnitts (224, 324) und des proximalen Spitzenabschnitts (222, 322) beträgt; oder wobei die kombinierte Fertiglänge des distalen Spitzenabschnitts (224, 324) und des proximalen Spitzenabschnitts (222, 322) ungefähr 2 cm beträgt, wobei der proximale Spitzenabschnitt (222, 322) eine Fertiglänge von ungefähr 1 cm hat, und der distale Spitzenabschnitt (224, 324) eine Fertiglänge von ungefähr 1 cm hat.

5. Verfahren zum Herstellen eines Spitzenabschnitts (218, 318) eines Führungsdrahtes (200, 300), welches Verfahren umfasst:
Durchführen des anfänglichen Ziehens eines Nitinoldrahtes, wobei der Draht eine Länge hat, um sowohl einen distalen Spitzenabschnitt (224, 324) als auch einen proximalen Spitzenabschnitt (222, 322) des Führungsdrahtes (200, 300) zu definieren, wobei das anfängliche Ziehen mindestens dem distalen Spitzenabschnitt (224, 324) eine ausreichende Kaltverarbeitung verleiht, um eher lineare elastische als superelastische Eigenschaften innerhalb des distalen Spitzenabschnitts (224, 324) bereitzustellen;
Wärmebehandlung mindestens des proximalen Spitzenabschnitts (222, 322) des Drahtes, um sicherzustellen, dass superelastische Eigenschaften innerhalb des proximalen Spitzenabschnitts (222, 322) bereitgestellt werden, ohne dem distalen Spitzenabschnitt (224, 324) superelastische Eigenschaften zu verleihen, so dass der proximale Spitzenabschnitt (222, 322) superelastische Eigenschaften aufweist, und der distale Spitzenabschnitt (224, 324) lineare elastische Eigenschaften aufweist;
**dadurch gekennzeichnet, dass** das Verfahren ferner das Schleifen oder anderweitiges Reduzieren einer Querschnittsdicke des Spitzenabschnitts (218, 318) umfasst, um einen kreisförmigen Querschnitt bereitzustellen, der einen gewünschten im Wesentlichen konstanten Durchmesser entlang des distalen Spitzenabschnitts (224, 324) und proximalen Spitzenabschnitts (222, 322) der Spitze hat.

6. Verfahren nach Anspruch 5, wobei das anfängliche Ziehen sowohl den proximalen (222, 322) als auch distalen (224, 324) Spitzenabschnitt linear elastisch macht, wobei die Wärmbehandlung des proximalen Spitzenabschnitts (222, 322) dem proximalen Spitzenabschnitt (222, 322) superelastische Eigenschaften verleiht, ohne dem distalen Spitzenabschnitt (224, 324) superelastische Eigenschaften zu verleihen; oder wobei der distale Spitzenabschnitt (224, 324) eine Fertiglänge hat, die ungefähr 50 % einer kombinierten Fertiglänge des distalen Spitzenabschnitts (224, 324) und des proximalen Spitzenabschnitts (222, 322) beträgt; oder wobei die kombinierte Fertiglänge des distalen Spitzenabschnitts (224, 324) und des proximalen Spitzenabschnitts (222, 322) ungefähr 2 cm beträgt, wobei der proximale Spitzenabschnitt (222, 322) eine Fertiglänge von ungefähr 1 cm hat, und der distale Spitzenabschnitt (224, 324) eine Fertiglänge von ungefähr 1 cm hat.

7. Führungsdraht (200, 300) umfassend:
einen Führungsdrahtspitzenabschnitt (218, 318) mit einem distalen Spitzenabschnitt (224, 324) und einem proximalen Spitzenabschnitt (222, 322), wobei der Spitzenabschnitt (218, 318) einen im Wesentlichen konstanten Durchmesser entlang sowohl des distalen Spitzenabschnitts (224, 324) als auch des proximalen Spitzenabschnitts (222, 322) mit einschließt;
wobei der distale Spitzenabschnitt (224, 324) einen kreisförmigen Querschnitt hat und eher lineare elastische als superelastische Eigenschaften aufweist; und
wobei der proximale Spitzenabschnitt (222, 322) einen kreisförmigen Querschnitt hat und superelastische Eigenschaft aufweist;
**dadurch gekennzeichnet, dass** der distale Spitzenabschnitt (224, 324) und der proximale Spitzenabschnitt (222, 322) einstückig aus einem einzigen Materialstück gebildet sind, um keine Verbindung dazwischen zu enthalten.

8. Führungsdraht (200, 300) nach Anspruch 7, wobei der distale Spitzenabschnitt (224, 324) eine Länge hat, die ungefähr 30 % bis ungefähr 70 % einer kombinierten Länge des distalen Spitzenabschnitts (224, 324) und des proximalen Spitzenabschnitts (222, 322) beträgt; oder wobei der distale Spitzenabschnitt (224, 324) eine Länge hat, die ungefähr 50 % einer kombinierten Länge des distalen Spitzenabschnitts (224, 324) und des proximalen Spitzenabschnitts (222, 322) beträgt; oder wobei eine kombinierte Länge des distalen Spitzenabschnitts (224, 324) und des proximalen Spitzenabschnitts (222, 322) ungefähr 2 cm beträgt, wobei der proximale Spitzenabschnitt (222, 322) eine Länge von ungefähr 1 cm hat, und der distale Spitzenabschnitt (224, 324) eine Länge von ungefähr 1 cm hat.

9. Führungsdraht (200, 300) nach Anspruch 7, wobei der Spitzenabschnitt (218, 318) im Durchschnitt mindestens 18 Windungen bis zum Versagen aufweist; oder wobei der Spitzenabschnitt (218, 318) im Durchschnitt mindestens 20 Windungen bis zum Versagen aufweist; oder wobei der Spitzenabschnitt (218, 318) im Durchschnitt mindestens 22 Windungen bis zum Versagen aufweist; oder wobei der Spitzenabschnitt (218, 318) eine Zunahme der durchschnittlichen Windungen bis zum Versagen von mindestens 15% im Vergleich zu einem ansonsten identischen Spitzenabschnitt (218, 318) aufweist, wobei der gesamte distale Spitzenabschnitt (224, 324) mit einem kreisförmigen und im Wesentlichen konstanten Querschnittsdurchmesser linear elastisch war; oder wobei der Spitzenabschnitt (218, 318) eine Zunahme der durchschnittlichen Windungen bis zum Versagen von mindestens 30% im Vergleich zu einem ansonsten identischen Spitzenabschnitt (218, 318) aufweist, wobei der gesamte distale Spitzenabschnitt (224, 324) mit einem kreisförmigen und im Wesentlichen konstanten Querschnittsdurchmesser linear elastisch war.

10. Verfahren zum Herstellen eines Kernmetallelements für einen medizinischen Führungsdraht (200, 300) umfassend:
Bereitstellung eines Drahtes (112a) aus Nickeltitanlegierung mit einer Länge, die einen proximalen Abschnitt (116) mit einem ersten Durchmesser und einen distalen Abschnitt mit einem zweiten Durchmesser mit einschließt;
Anwendung von Kaltarbeit auf den distalen Abschnitt (118) und Nichtanwendung von Kaltarbeit auf den proximalen Abschnitt (116), wodurch dem distalen Abschnitt (118) ein dritter Durchmesser, der kleiner als der zweite Durchmesser ist, verliehen wird; und dann
Anwendung eines Reduktionsprozesses auf den Draht, wodurch der proximale Abschnitt (116) reduziert wird, damit er einen vierten Durchmesser erhält, der kleiner als der erste Durchmesser ist;
**dadurch gekennzeichnet, dass** der Reduktionsprozess zu einem kreisförmigen Querschnitt entlang des distalen Abschnitts (118) und proximalen Abschnitts (116) führt, der einen im Wesentlichen konstanten Durchmesser hat.

11. Verfahren nach Anspruch 10, wobei die Bereitstellung eines Drahtes (112a) die Bereitstellung eines Drahtes (112a) mit superelastischen Eigenschaften über die Länge umfasst; oder wobei die Bereitstellung eines Drahtes (112a) die Bereitstellung eines Drahtes (112a) umfasst, wobei der proximale Abschnitt (116) dem distalen Abschnitt (118) benachbart ist; oder wobei die Bereitstellung eines Drahtes (112a) die Bereitstellung eines Drahtes (112a) umfasst, wobei der proximale Abschnitt (116) einem proximalen Ende des Drahtes (112a) benachbart ist; oder wobei die Bereitstellung eines Drahtes (112a) die Bereitstellung eines Drahtes (112a) umfasst, wobei der distale Abschnitt (118) einem distalen Ende des Drahtes (112a) benachbart ist.

12. Verfahren nach Anspruch 10, wobei die Anwendung von Kaltarbeit auf den distalen Abschnitt (118) die Anwendung einer ausreichenden Kaltarbeit umfasst, um den distalen Abschnitt (118) mit linearen elastischen Eigenschaften zu versehen, bevorzugt wobei nach der Anwendung von Kaltarbeit auf den distalen Abschnitt (118) der proximale Abschnitt (116) superelastische Eigenschaften beibehält, eher bevorzugt wobei keine Verbindung in den Draht (112a) über die Länge eingeführt wird; oder eher bevorzugt kein Schweißprozess auf den Draht (112a) über die Länge angewendet wird, am meisten bevorzugt ferner umfassend das Entfernen des Führungsdrahtes (200, 300) von der Matrize ohne Zurückziehen des distalen Abschnitts (118) durch die Matrize.

13. Verfahren nach Anspruch 10, wobei die Anwendung eines Reduktionsprozesses auf den Führungsdraht (200, 300) die Anwendung von Centerless-Schleifen umfasst; oder wobei die Bereitstellung eines Drahtes (112a) die Bereitstellung eines Drahtes (112a) umfasst, wobei der erste Durchmesser der gleiche wie der zweite Durchmesser ist; oder wobei die Anwendung eines Reduktionsprozesses auf den Draht (112a) die Reduktion des proximalen Abschnitts (116) umfasst, damit er einen vierten Durchmesser erhält, welcher der gleiche wie der dritte Durchmesser ist; oder wobei die Anwendung eines Reduktionsprozesses auf den Draht (112a) die Reduktion des distalen Abschnitts (118) umfasst, damit er einen fünften Durchmesser erhält, der kleiner als der dritte Durchmesser ist; oder wobei die Anwendung von Kaltarbeit auf den distalen Abschnitt (118) das Ziehen des distalen Abschnitts (118) durch eine Matrize umfasst, bevorzugt wobei die Anwendung von Kaltarbeit auf den distalen Abschnitt (118) die Anwendung von Kaltarbeitsverfahren umfasst, die ausgewählt sind aus: Rundkneten, Rollen, Verspannung, Stanzen, Prägung und Kombination davon.

14. Medizinischer Führungsdraht (200, 300) umfassend:
einen massiven Metallkern (112) mit einer Länge und mit einem konstanten Durchmesser über die Länge, wobei die Länge einen proximalen Abschnitt (116) mit pseudoelastischen Eigenschaften und einen distalen Abschnitt (118) mit linearen elastischen Eigenschaften umfasst, und wobei die Länge des Kerns (112) keine mechanische Verbindung an einer beliebigen Stelle zwischen dem proximalen Abschnitt (116) und dem distalen Abschnitt (118) beinhaltet.

15. Führungsdraht nach Anspruch 14, wobei der proximale Abschnitt (116) aus einer Nickeltitanlegierung gebildet ist; oder wobei der distale Abschnitt (118) Metall enthält, dem die linearen elastischen Eigenschaften durch einen Kaltverarbeitungsprozess verliehen wurden.

## Revendications

1. Procédé de fabrication d'une partie de pointe (218, 318) d'un fil de guidage (200, 300), le procédé comprenant :
la fourniture d'un fil superélastique (202, 302) comprenant une longueur de manière à définir à la fois une partie de pointe distale (224, 324) et une partie de pointe proximale (222, 322) ;
le travail au froid de la partie de pointe distale (224, 324) sans conférer un travail à froid significatif à la partie de pointe proximale (222, 322) pour fournir des propriétés élastiques linéaires plutôt que superélastiques à l'intérieur de la partie de pointe distale (224, 324), si bien que la partie de pointe proximale (222, 322) présente des propriétés superélastiques et la partie de pointe distale (224, 324) présente des propriétés élastiques linéaires ; et
le broyage ou une autre réduction de l'épaisseur de la section transversale de la partie de pointe (218, 318) après le travail à froid pour fournir une section transversale circulaire ayant un diamètre essentiellement constant souhaité le long de la partie de pointe distale et de la partie de pointe proximale (222, 322) de la pointe.

2. Procédé selon la revendication 1, dans lequel le travail à froid de la partie de pointe distale (224, 324) comprend un rétreint rotatif de la partie de pointe distale (224, 324) sans conférer un travail à froid significatif à la partie de pointe proximale (222, 322) ; de préférence, dans lequel le rétreint rotatif réduit un diamètre de la partie de pointe distale (224, 324) d'au plus environ 15%, ou dans lequel le rétreint rotatif réduit un diamètre de la partie de pointe distale (224, 324) d'environ 5% à environ 15%.

3. Procédé de fabrication d'une partie de pointe (218, 318) d'un fil de guidage (200, 300), le procédé comprenant :
la fourniture d'un fil superélastique (202, 302) comprenant une longueur de manière à définir à la fois une partie de pointe distale (224, 324) et une partie de pointe proximale (222, 322) ;
le rétreint rotatif de la partie de pointe distale (224, 324) sans conférer un travail à froid significatif à la partie de pointe proximale (222, 322) pour fournir des propriétés élastiques linéaires plutôt que superélastiques à l'intérieur de la partie de pointe distale (224, 324), si bien que la partie de pointe proximale (222, 322) présente des propriétés superélastiques et la partie de pointe distale (224, 324) présente des propriétés élastiques linéaires ; et
le broyage de la partie de pointe (218, 318) après le travail à froid pour fournir une section transversale circulaire ayant un diamètre essentiellement constant souhaité le long de la partie de pointe distale (224, 324) et de la partie de pointe proximale (222, 322) de la pointe;
dans lequel la partie de pointe distale (224, 324) présente une longueur finie qui est d'environ 30% à environ 70% de celle d'une longueur finie combinée de la partie de pointe distale (224, 324) et de la partie de pointe proximale (222, 322).

4. Procédé selon la revendication 3, dans lequel le rétreint rotatif réduit un diamètre de la partie de pointe distale (224, 324) d'au plus environ 15% ; dans lequel le rétreint rotatif réduit un diamètre de la partie de pointe distale (224, 324) d'environ 5% à environ 15% ; ou dans lequel la partie de pointe distale (224, 324) présente une longueur finie qui est d'environ 50% de celle d'une longueur finie combinée de la partie de pointe distale (224, 324) et de la partie de pointe proximale (222, 322) ; ou dans lequel la longueur finie combinée de la partie de pointe distale (224, 324) et de la partie de pointe proximale (222, 322) est environ 2 cm, la partie de pointe proximale (222, 322) ayant une longueur finie d'environ 1 cm et la partie de pointe distale (224, 324) ayant une longueur finie d'environ 1 cm.

5. Procédé de fabrication d'une partie de pointe (218, 318) d'un fil de guidage (200, 300), le procédé comprenant :
l'exécution d'un étirage initial d'un fil de nitinol, le fil ayant une longueur de manière à définir à la fois une partie de pointe distale (224, 324) et une partie de pointe proximale (222, 322) du fil de guidage (200, 300), l'étirage initial conférant un travail à froid suffisant à au moins la partie de pointe distale (224, 324) pour fournir des propriétés élastiques linéaires plutôt que superélastiques à l'intérieur de la partie de pointe distale (224, 324) ;
le traitement thermique de la partie de pointe proximale (222, 322) du fil pour garantir que des propriétés superélastiques sont fournies à l'intérieur de la partie de pointe proximale (222, 322) sans conférer des propriétés superélastiques à la partie de pointe distale (224, 324) si bien que la partie de pointe proximale (222, 322) présente des propriétés superélastiques, et la partie de pointe distale (224, 324) présente des propriétés élastiques linéaires ;
**caractérisé en ce que** le procédé comprend en outre le broyage ou une autre réduction de l'épaisseur d'une section transversale de la partie de pointe (218, 318) pour fournir une section transversale circulaire ayant un diamètre essentiellement constant souhaité le long de la partie de pointe distale (224, 324) et de la partie de pointe proximale (222, 322) de la pointe.

6. Procédé selon la revendication 5, dans lequel l'étirage initial rend les parties de pointe proximale (222, 322) et distale (224, 324) élastiques linéaires, le traitement thermique de la partie de pointe proximale (222, 322) conférant des propriétés superélastiques à la partie de pointe proximale (222, 322) sans conférer des propriétés superélastiques à la partie de pointe distale (224, 324) ; ou dans lequel la partie de pointe distale (224, 324) présente une longueur finie qui est d'environ 50% de celle d'une longueur finie combinée de la partie de pointe distale (224, 324) et de la partie de pointe proximale (222, 322) ; ou dans lequel la longueur finie combinée de la partie de pointe distale (224, 324) et de la partie de pointe proximale (222, 322) est environ 2 cm, la partie de pointe proximale (222, 322) ayant une longueur finie d'environ 1 cm et la partie de pointe distale (224, 324) ayant une longueur finie d'environ 1 cm.

7. Fil de guidage (200, 300) comprenant :
une partie de pointe de fil de guidage (218, 318) comprenant une partie de pointe distale (224, 324) et une partie de pointe proximale (222, 322), la partie de pointe (218, 318) comprenant un diamètre essentiellement constant le long à la fois de la partie de pointe distale (224, 324) et de la partie de pointe proximale (222, 322) ;
la partie de pointe distale (224, 324) ayant une section transversale circulaire et présentant des propriétés élastiques linéaires plutôt que superélastiques ; et
la partie de pointe proximale (222, 322) ayant une section transversale circulaire et présentant des propriétés superélastiques ;
**caractérisé en ce que** la partie de pointe distale (224, 324) et la partie de pointe proximale (222, 322) sont intégralement formées à partir d'une seule pièce de matériau de manière à ne pas inclure de joint entre elles.

8. Fil de guidage (200, 300) selon la revendication 7, dans lequel la partie de pointe distale (224, 324) présente une longueur qui est d'environ 30% à environ 70% de celle d'une longueur combinée de la partie de pointe distale (224, 324) et de la partie de pointe proximale (222, 322); ou dans lequel la partie de pointe distale (224, 324) présente une longueur qui est d'environ 50% de celle d'une longueur combinée de la partie de pointe distale (224, 324) et de la partie de pointe proximale (222, 322) ; ou dans lequel une longueur combinée de la partie de pointe distale (224, 324) et de la partie de pointe proximale (222, 322) est environ 2 cm, la partie de pointe proximale (222, 322) ayant une longueur d'environ 1 cm et la partie de pointe distale (224, 324) ayant une longueur d'environ 1 cm.

9. Fil de guidage (200, 300) selon la revendication 7, dans lequel la partie de pointe (218, 318) présente au moins 18 tours à défaillance en moyenne ; ou dans lequel la partie de pointe (218, 318) présente au moins 20 tours à défaillance en moyenne ; ou dans lequel la partie de pointe (218, 318) présente au moins 22 tours à défaillance en moyenne ; ou dans lequel la partie de pointe (218, 318) présente une augmentation d'au moins 15% des tours à défaillance en moyenne par rapport à une partie de pointe (218, 318) autrement identique où dans lequel la totalité de la partie de pointe distale (224, 324) ayant une section transversale circulaire et un diamètre essentiellement constant était élastique linéaire ; ou dans lequel la partie de pointe (218, 318) présente une augmentation d'au moins 30% des tours à défaillance en moyenne par rapport à une partie de pointe (218, 318) autrement identique dans lequel la totalité de la partie de pointe distale (224, 324) ayant une section transversale circulaire et un diamètre essentiellement constant était élastique linéaire.

10. Procédé de fabrication d'un élément métallique central pour un fil de guidage médical (200, 300) comprenant :
la fourniture d'un fil (112a) en alliage nickel-titane ayant une longueur qui comprend une partie proximale (116) ayant un premier diamètre et une partie distale ayant un deuxième diamètre ;
l'application d'un travail à froid sur la partie distale (118) et le fait de ne pas appliquer de travail à froid sur la partie proximale (116), conférant ainsi à la partie distale (118) un troisième diamètre inférieur au deuxième diamètre ; et puis
l'application d'un processus de réduction au fil, par lequel la partie proximale (116) est réduite pour avoir un quatrième diamètre qui est inférieur au premier diamètre ;
**caractérisé en ce que** le processus de réduction aboutit à une section transversale circulaire le long de la partie de pointe distale (118) et de la partie de pointe proximale (116) dont le diamètre est essentiellement constant.

11. Procédé selon la revendication 10, dans lequel la fourniture d'un fil (112a) comprend la fourniture d'un fil (112a) avec des propriétés superélastiques sur toute la longueur ; ou dans lequel la fourniture d'un fil (112a) comprend la fourniture d'un fil (112a) où la partie proximale (116) est adjacente à la partie distale (118); ou dans lequel la fourniture d'un fil (112a) comprend la fourniture d'un fil (112a) où la partie proximale (116) est adjacente à une extrémité proximale du fil (112a) ; ou dans lequel la fourniture d'un fil (112a) comprend la fourniture d'un fil (112a) où la partie distale (118) est adjacente à une extrémité distale du fil (112a).

12. Procédé selon la revendication 10, dans lequel l'application d'un travail à froid sur la partie distale (118) comprend l'application d'un travail à froid suffisant pour rendre la partie distale (118) ayant des propriétés élastiques linéaires, de préférence dans lequel après avoir appliqué un travail à froid sur la partie distale (118), la partie proximale (116) conserve des propriétés superélastiques, plus préférablement dans lequel aucun joint n'est inséré dans le fil (112a) sur la longueur ; ou plus préférablement dans lequel aucun processus de soudure n'est appliqué au fil (112a) sur la longueur, plus préférablement comprenant en outre le retrait du fil de guidage (200, 300) à partir de la matrice sans tirer la partie distale (118) en arrière à travers la matrice.

13. Procédé selon la revendication 10, dans lequel l'application d'un processus de réduction au fil de guidage (200, 300) comprend l'application d'un broyage sans centre ; ou dans lequel la fourniture d'un fil (112a) comprend la fourniture d'un fil (112a) dans lequel le premier diamètre est le même que le deuxième diamètre ; ou dans lequel l'application d'un processus de réduction au fil (112a) comprend la réduction de la partie proximale (116) pour avoir un quatrième diamètre qui est le même que le troisième diamètre ; ou dans lequel l'application d'un processus de réduction au fil (112a) comprend la réduction de la partie distale (118) pour avoir un cinquième diamètre qui est inférieur au troisième diamètre ; ou dans lequel l'application d'un travail à froid sur la partie distale (118) comprend l'étirage de la partie distale (118) à travers une matrice, de préférence l'application d'un travail à froid sur la partie distale (118) comprend l'application de méthodes de travail à froid choisies parmi: emboutissage (swaging), roulage, tension, estampage, frappe et leurs combinaisons.

14. Fil de guidage médical (200, 300) comprenant :
un noyau métallique solide (112) ayant une longueur et un diamètre constant sur la longueur, dans lequel la longueur comprend une partie proximale (116) ayant des propriétés pseudo-élastiques et une partie distale (118) ayant des propriétés élastiques linéaires, et dans lequel la longueur du noyau (112) ne comprend pas un joint mécanique à n'importe quel emplacement entre la partie proximale (116) et la partie distale (118).

15. Fil de guidage selon la revendication 14, dans lequel la partie proximale (116) est formée d'un alliage nickel-titane ; ou dans lequel la partie distale (118) comprend du métal auquel les propriétés élastiques linéaires ont été conférées par un processus de travail à froid.
